(19) Europäisches Patentamt
European Patent Office
Office européen des brevets

(11) **EP 4 414 013 B1**

(12) **EUROPÄISCHE PATENTSCHRIFT**

(45) Veröffentlichungstag und Bekanntmachung des Hinweises auf die Patenterteilung:
**01.04.2026 Patentblatt 2026/14**

(21) Anmeldenummer: **24154925.2**

(22) Anmeldetag: **31.01.2024**

(51) Internationale Patentklassifikation (IPC):
***A61M 16/08*** *(2006.01)*

(52) Gemeinsame Patentklassifikation (CPC):
(C-Sets verfügbar)
**A61M 16/12; A61M 16/024; A61M 16/0891; A61M 16/104;** A61M 16/0051; A61M 16/0066; A61M 16/0078; A61M 16/01; A61M 16/0816; A61M 16/18; A61M 16/201; A61M 16/22; A61M 2016/0039; A61M 2016/0042; A61M 2202/0208; (Forts.)

(54) **BEATMUNGSSYSTEM ZUR KÜNSTLICHEN BEATMUNG MIT EINEM ANZEIGEELEMENT FÜR EINEN VOLUMENFLUSS**

VENTILATION SYSTEM FOR ARTIFICIAL RESPIRATION COMPRISING A DISPLAY ELEMENT FOR A VOLUME FLOW

SYSTÈME DE VENTILATION ARTIFICIELLE AVEC UN ÉLÉMENT D'AFFICHAGE POUR UN DÉBIT VOLUMIQUE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **07.02.2023 DE 102023102932**

(43) Veröffentlichungstag der Anmeldung:
**14.08.2024 Patentblatt 2024/33**

(73) Patentinhaber: **Drägerwerk AG & Co. KGaA 23558 Lübeck (DE)**

(72) Erfinder:
• **Schmid, Robert 23558 Lübeck (DE)**
• **Wagener, Guido 23558 Lübeck (DE)**

(56) Entgegenhaltungen:
WO-A1-2021/189138 DE-A1- 102008 028 662
DE-A1- 102013 208 761 US-A1- 2009 143 996
US-A1- 2010 242 622 US-A1- 2019 099 578
US-A1- 2022 080 139

**(Forts. nächste Seite)**

(52) Gemeinsame Patentklassifikation (CPC): (Forts.)
A61M 2202/0225; A61M 2202/0283; A61M 2205/18;
A61M 2205/3334; A61M 2205/502; A61M 2205/581;
A61M 2205/582; A61M 2205/583; A61M 2205/587;
A61M 2230/432

C-Sets
A61M 2202/0208, A61M 2202/0007;
A61M 2202/0225, A61M 2202/0021;
A61M 2202/0283, A61M 2202/0007

**Beschreibung**

Die Erfindung betrifft ein Beatmungssystem, welches einen Patienten künstlich zu beatmen vermag und welches mindestens ein Anzeigeelement umfasst, das die Fließrichtung eines Gases anzeigt.

Ein Beatmungssystem, welches einen Patienten künstlich zu beatmen vermag, umfasst üblicherweise ein Beatmungsgerät, eine patientenseitige Koppeleinheit und eine Inspirations-Fluidführungseinheit. Die patientenseitige Koppeleinheit ist in und / oder an und / oder auf dem Körper des Patienten angeordnet und umfasst beispielsweise eine Atemmaske und / oder einen Tubus und/oder einen Katheter. Die Inspirations-Fluidführungseinheit vermag ein Fluid vom Beatmungsgerät zur patientenseitigen Koppeleinheit zu leiten und umfasst beispielsweise einen Schlauch und optional zusätzlich eine Röhre. Das Beatmungsgerät führt eine Abfolge von Beatmungshüben aus, wobei das Beatmungsgerät in jedem Beatmungshub jeweils eine Menge eines Gasgemischs ausstößt und die ausgestoßene Menge vom Beatmungsgerät durch die Inspirations-Fluidführungseinheit hindurch zur patientenseitigen Koppeleinheit geleitet wird. Das Gasgemisch umfasst Sauerstoff. Auch das erfindungsgemäße Beatmungssystem verwendet dieses Prinzip.

In einer Ausgestaltung umfasst das Gasgemisch zusätzlich mindestens ein Narkosemittel, und der Patient ist daher sediert oder narkotisiert. Insbesondere in diesem Fall realisiert das Beatmungssystem in der Regel einen Beatmungskreislauf. Das Beatmungsgerät und optional die eigene Atmungsaktivität des Patienten halten diesen Beatmungskreislauf aufrecht. Das vom Patienten ausgeatmete Gasgemisch enthält in der Regel Narkosemittel und wird von der patientenseitigen Koppeleinheit durch eine Exspirations-Fluidführungseinheit hindurch zurück zum Beatmungsgerät geleitet. Dank des Beatmungskreislaufs gerät dieses Narkosemittel nicht in die Umgebung. In einer Anwendung verwendet auch das erfindungsgemäße Beatmungssystem dieses Prinzip.

Gewünscht wird, dass ein Anästhesist oder ein sonstiger Benutzer rasch feststellen kann, ob der Patient tatsächlich wie gewünscht künstlich beatmet wird oder ob ein Fehler aufgetreten ist.

Aus dem Stand der Technik sind beispielsweise folgende Vorrichtungen bekannt: US 2010/242622 A1 offenbart einen Durchflusssensor, welcher über ein Messgerät mit einem Analysegerät verbunden ist, wobei in dem Analysegerät das gemessene Signal so verarbeitet wird, dass eine Anzeige der Durchfluss- und Druckwerte für Ein- und Ausatmung und anderer davon abgeleiteter Größen erzeugt wird. Optional kann die Messvorrichtung im Analysegerät untergebracht sein.

US 2019/099578 A1 offenbart einen Atemadapter mit einem Pneumotachometer.

DE 10 2008 028 662 A1 offenbart eine Vorrichtung zum Messen und Signalisieren zumindest eines Beatmungsparameters und/oder eines physiologischen Parameters, wobei die Vorrichtung lösbar im Bereich einer Beatmungsvorrichtung angeordnet werden kann.

US 2022/080139 A1 offenbart eine Vorrichtung zum Bestimmen von Informationen, die sich auf Adhärenz eines Patienten mit einer Störung der Atemwege beziehen, umfassend einen Fluidkanal zum Leiten von einem medizinischen Gas und eine Sensoranordnung mit Sensoren zum Messen der Strömungsparameterwerte in dem Kanal, wobei die Vorrichtung dazu ausgebildet ist um die Strömungsrichtung des medizinischen Gases in dem Kanal unter Verwendung einer Steuereinheit zu bestimmen und die Kupplungsausrichtung der Vorrichtung anzuzeigen.

US 2009/143996 A1 offenbart einen Atemwegssensor, bestehend aus einem Gasanalysegerät zur Analyse mindestens eines Atemgases und einer Verbindungseinheit zur Kommunikation mit einem entfernt gelegenen Hostgerät, wobei das Gasanalysegerät mit einer Sensorelektronik verbunden ist und diese Sensorelektronik zur Messung des Atemgasdurchflusses und des Atemwegsdrucks genutzt werden kann.

Der Erfindung liegt die Aufgabe zugrunde, ein Beatmungssystem bereitzustellen, welches es in vielen Fällen einem Benutzer erleichtert, das Beatmungssystem zu überwachen.

Die Aufgabe wird durch ein Beatmungssystem mit den Merkmalen des Anspruchs 1 gelöst. Die Unteransprüche beschreiben vorteilhafte Ausgestaltungen des erfindungsgemäßen Beatmungssystems.

Das erfindungsgemäße Beatmungssystem umfasst ein Beatmungsgerät (Ventilator). Das Beatmungsgerät vermag ein Gasgemisch auszustoßen. Dieses Gasgemisch umfasst Sauerstoff. In einer Ausgestaltung ist das Gasgemisch reiner Sauerstoff. In einer bevorzugten Ausgestaltung umfasst das Gasgemisch mindestens einen weiteren Gas- Bestandteil, beispielsweise Atemluft und / oder mindestens ein Narkosemittel. Ein Anästhesiegerät ist eine mögliche Ausgestaltung eines Beatmungsgeräts im Sinne der Ansprüche. Bevorzugt führt das Beatmungsgerät eine Abfolge von Beatmungshüben durch und stößt in jedem Beatmungshub jeweils eine Menge des Gasgemischs aus.

Weiterhin umfasst das Beatmungssystem eine patientenseitige Koppeleinheit. Die patientenseitige Koppeleinheit ist wenigstens zeitweise mit dem Patienten verbunden oder lässt sich mit ihm verbinden. Eine Atemmaske, ein Tubus und ein Katheter sind Ausgestaltungen und/oder mögliche Bestandteile der patientenseitige Koppeleinheit.

Eine Inspirations-Fluidführungseinheit des Beatmungssystems vermag ein Gasgemisch, welches das Beatmungsgerät ausgestoßen hat, zur patientenseitigen Koppeleinheit zu leiten. Unter einer «Fluidführungseinheit" wird ein Bauteil verstanden, welches ein Fluid entlang einer durch die Konstruktion und Anordnung des Bauteils vorgegebenen Trajektorie zu leiten vermag und idealerweise verhindert, dass das Fluid diese Trajektorie verlässt. Ein Schlauch und eine Röhre sind zwei Beispiele für eine Fluidführungseinheit.

Das Beatmungssystem umfasst weiterhin ein Inspirations-Anzeigeelement. Unter einem "Anzeigeelement" wird ein ansteuerbares Bauteil verstanden, welches eine Information über das Beatmungssystem in mindestens einer visuell von einem Menschen wahrnehmbaren Weise auszugeben vermag. In einer ersten Alternative ist dieses Inspirations-Anzeigeelement an der Inspirations-Fluidführungseinheit befestigt. In einer zweiten Alternative ist das Inspirations-Anzeigeelement an einer anderen Fluidführungseinheit befestigt, wobei diese andere Fluidführungseinheit in einer Fluidverbindung mit dem Beatmungsgerät und mit der Inspirations-Fluidführungseinheit steht. Zwischen zwei Bauteilen ist eine "Fluidverbindung" hergestellt, wenn ein Fluid wenigstens zeitweise von dem einen Bauteil zu dem anderen Bauteil fließen kann.

Ein signalverarbeitendes Steuergerät (control unit) des Beatmungssystems vermag ein Maß für den Netto-Inspirations-Volumenfluss zu ermitteln. Unter einem "Volumenfluss" (Volumenstrom) durch eine Fluidführungseinheit wird das Volumen pro Zeiteinheit verstanden, das durch diese Fluidführungseinheit fließt. In der Regel variiert der Volumenfluss über die Zeit. Natürlich ist es möglich, dass wenigstens zeitweise kein Fluid durch die Fluidführungseinheit fließt und der Volumenfluss daher gleich Null ist oder wenigstens unterhalb einer vorgegebenen unteren Schranke liegt.

Unter dem "Netto-Inspirations-Volumenfluss" wird derjenige Volumenfluss durch die Inspirations-Fluidführungseinheit hindurch verstanden, den das Beatmungsgerät erzeugt, bevorzugt mit seinen Beatmungshüben. Der gesamte Volumenfluss durch die Inspirations-Fluidführungseinheit hindurch kann eine Überlagerung des Netto-Inspirations-Volumenflusses und mindestens eines weiteren Volumenflusses sein. Ein weiterer Volumenfluss kann insbesondere dadurch erzeugt werden, dass ein Beatmungskreislauf hergestellt ist, so dass ein Exspirations-Gasgemisch von der patientenseitigen Koppeleinheit und durch das Beatmungsgerät hindurch zurück in die Inspirations-Fluidführungseinheit fließt. Durch die Inspirations-Fluidführungseinheit hindurch kann zusätzlich ein Fluid fließen, welches verwendet wird, um eine Fluidführungseinheit zu reinigen oder um die Konzentration eines Gas-Bestandteils in einem Gasgemisch zu messen. Außerdem kann ein Leck in der Inspirations-Fluidführungseinheit zu einem Volumenfluss führen, der nicht die patientenseitige Koppeleinheit erreicht. Möglich ist auch, dass gesamte Volumenfluss durch die Inspirations-Fluidführungseinheit gleich dem Netto-Inspirations-Volumenfluss ist.

Das Steuergerät vermag das Inspirations-Anzeigeelement abhängig vom ermittelten Netto-Inspirations-Volumenfluss anzusteuern. Abhängig von der Ansteuerung vermag das Inspirations-Anzeigeelement mindestens zwei Indikatoren und dadurch die folgenden beiden Informationen darzustellen, und zwar auf mindestens eine Weise, die ein Mensch visuell wahrnehmen kann:

- einen Indikator für die Größe (den Betrag) ermittelten Netto-Inspirations-Volumenflusses und

- einen Indikator für die Fließrichtung des Gasgemischs durch die Inspirations-Fluidführungseinheit hindurch.

Die beiden Indikatoren unterscheiden sich voneinander in mindestens einer visuell wahrnehmbaren Weise. Dadurch lässt sich unterscheiden, welcher Indikator sich auf den Netto-Inspirations-Volumenfluss und welcher sich auf die Fließrichtung bezieht. Die beiden Indikatoren beschreiben zwei verschiedene Zustände des Beatmungssystems.

Im einfachsten Fall zeigt der Indikator für den Netto-Inspirations-Volumenfluss an, ob durch die Inspirations-Fluidführungseinheit ein Fluid mit einem Volumenfluss oberhalb einer vorgegebenen unteren Schranke fließt oder nicht. Möglich ist auch, dass dieser Indikator zusätzlich ein kontinuierliches oder gestuftes Maß für die Größe (den Betrag) des Volumenflusses anzeigt.

Der Indikator für die Fließrichtung zeigt an, ob Fluid vom Beatmungsgerät durch die Inspirations-Fluidführungseinheit hindurch zur patientenseitigen Koppeleinheit fließt oder in die entgegengesetzte Richtung. Falls kein Netto-Inspirations-Volumenfluss oberhalb einer vorgegebenen unteren Schranke auftritt, so wird bevorzugt ein anderer oder überhaupt kein Indikator für die Fließrichtung angezeigt.

Während einer künstlichen Beatmung können verschiedene Fehler auftreten. Diese Fehler können einen künstlich beatmeten Patienten gefährden. Wichtig ist daher, dass ein Benutzer diese Fehler rasch erkennt. Beispiele für solche Fehler, die ein Benutzer aufgrund der Erfindung erkennen und in vielen Fällen auch beseitigen kann, sind die folgenden:

- Fälschlicherweise wurde eine Fluidverbindung zwischen dem Beatmungsgerät und der patientenseitigen Koppeleinheit nicht hergestellt.
- Eine Fluidführungseinheit ist nicht mit dem richtigen Anschlusselement am Beatmungsgerät verbunden, sondern mit einem falschen.
- Weil eine Fluidführungseinheit mit einem zu kleinen Durchmesser verwendet wird, ist der erzielte Volumenfluss kleiner als der erforderliche Volumenfluss.
- Eine Fluidführungseinheit oder die patientenseitige Koppeleinheit sind geknickt oder verstopft oder fälschlicherweise verschlossen, so dass der tatsächliche Volumenfluss geringer als der erforderliche Volumenfluss ist oder fälschlicherweise sogar überhaupt kein Fluid fließt.

- Eine Fluidführungseinheit ist nicht fluiddicht mit dem Beatmungsgerät verbunden, so dass ein Teil des Gasgemischs, das bei einem Beatmungshub ausgestoßen wird, in die Umgebung gelangt, anstelle zur patientenseitigen Koppeleinheit geleitet zu werden.
- Ein Leck an einer Fluidführungseinheit oder ein verstopfter Filter führen zu einem zu großen oder auch zu kleinen Volumenfluss, und der Volumenfluss zur patientenseitigen Koppeleinheit ist daher zu klein.
- Das Beatmungsgerät vermag nicht die erforderliche Menge des Gasgemischs auszustoßen, sondern eine zu geringe Menge oder überhaupt kein Gasgemisch.
- Durch eine falsche Benutzervorgabe ist am Beatmungsgerät eine falsche Vorgabe für einen Volumenfluss eingestellt.

Die Erfindung bewirkt, dass in einer ergonomischen Weise der aktuelle interne Zustand einer Vorrichtung angezeigt wird, nämlich ein Zustand des Beatmungssystems.

Denkbar wäre es, auf einer zentralen Anzeigeeinheit den jeweiligen Volumenfluss durch mehrere Fluidführungseinheiten eines Beatmungssystems anzuzeigen. Die Erfindung lässt sich mit einer solchen zentralen Anzeigeeinheit kombinieren. Diese Ausgestaltung erfordert aber, auf der zentralen Anzeigeeinheit zusätzlich anzuzeigen, welcher ausgegebene (dargestellte) Wert sich auf welche Fluidführungseinheit bezieht. Diese Information könnte textlich und / oder graphisch ausgegeben werden. Eine Ausgabe auf einer zentralen Ausgabeeinheit erfordert mehr Platz und in manchen Fällen mehr Rechenkapazität als die erfindungsgemäße Ausgabe von Informationen. Gerade in der klinischen Praxis ist häufig der Platz begrenzt, der für eine zentrale Anzeigeeinheit zur Verfügung steht. Außerdem erfordert die Verwendung einer zentralen Ausgabeeinheit, dass ein Benutzer versteht, welcher ausgegebene Wert für einen Volumenfluss sich auf welche Fluidführungseinheit bezieht.

Die Erfindung erspart hingegen die Notwendigkeit, eine zentrale Anzeigeeinheit verwenden zu müssen, um eine Information über einen Inspirations-Volumenfluss auszugeben, und spart dadurch Platz ein. Außerdem erspart die Erfindung die Notwendigkeit, dass ein Benutzer in einer zentralen Darstellung eine Darstellung einer Fluidführungseinheit mit der dargestellten realen (physikalischen) Fluidführungseinheit gedanklich verbinden muss. Erfindungsgemäß werden die Informationen über einen Volumenfluss durch eine Fluidführungseinheit hingegen direkt an dieser Fluidführungseinheit angezeigt. Dadurch reduziert die Erfindung die Gefahr von Fehlern, insbesondere die Gefahr, dass ein Benutzer eine Information nicht auffindet oder falsch zuordnet. Außerdem kann der Benutzer die erfindungsgemäß dargestellten Informationen häufig rascher erfassen.

Erfindungsgemäß zeigt das Inspirations-Anzeigeelement die beiden Indikatoren in jeweils einer visuell wahrnehmbaren Weise an. Möglich ist, dass bei einem falschen Volumenfluss zusätzlich ein Alarm akustisch und / oder taktil (haptisch), bevorzugt durch Vibrationen, ausgegeben und/oder auf einer räumlich entfernten Anzeigeeinheit ausgegeben wird. Das erfindungsgemäße Merkmal, dass das Inspirations-Anzeigeelement zwei Indikatoren visuell ausgibt, erspart aber die Notwendigkeit, Fehler ausschließlich durch akustische oder haptische Alarme und / oder Alarme auf einer Anzeigeeinheit eines räumlich entfernten Empfängers zu melden. In vielen Fällen zeigt ein akustischer oder haptischer Alarm nur an, dass eine Störung aufgetreten ist, aber nicht wo. Außerdem dringen im klinischen Alltag häufig viele Geräusche auf einen Benutzer ein. Ein räumlich entfernter Empfänger ist häufig relativ weit von dem Ort entfernt, an dem der Fehler aufgetreten ist. Die Erfindung erspart die Notwendigkeit, bei einem Fehler am Beatmungssystem einen Benutzer ausschließlich durch einen akustischen oder haptischen Alarm zu informieren. Vielmehr ermöglicht die Erfindung es, den Benutzer visuell und optional zusätzlich akustisch und / oder haptisch zu warnen.

Erfindungsgemäß zeigt das angesteuerte Inspirations-Anzeigeelement zwei verschiedene Indikatoren an, nämlich einen für den Netto-Inspirations-Volumenfluss und einen für die Fließrichtung. Die beiden Indikatoren lassen sich in einer visuell wahrnehmbaren Weise voneinander unterscheiden. Dieses Merkmal führt dazu, dass in vielen Fällen ein Benutzer zuverlässiger und / oder schneller die dargestellten Informationen über den Zustand des Beatmungssystems erfassen kann, als wenn derselbe Indikator sowohl den Netto-Inspirations-Volumenfluss als auch die Fließrichtung anzeigen würde.

Dies wird an einem Beispiel erläutert. Der Indikator für den Netto-Inspirations-Volumenfluss hat gemäß dem Beispiel drei mögliche Werte, beispielsweise grün, gelb und rot. Der Wert grün bedeutet einen Volumenfluss im Sollbereich, die Werte gelb und rot einen Volumenfluss außerhalb des Sollbereichs, wobei der Wert rot ein sofortiges Eingreifen erfordert. Der Indikator für die Fließrichtung hat ebenfalls drei mögliche Werte, beispielsweise einen Pfeil, der vom Beatmungsgerät zur patientenseitigen Koppeleinheit zeigt, einen Pfeil in die entgegengesetzte Richtung und ein Symbol dafür, dass kein Volumenfluss oberhalb einer vorgegebenen unteren Schranke auftritt.

Ein Benutzer kann die jeweils drei unterschiedliche Symbole rasch erfassen und voneinander unterscheiden und außerdem erfassen, ob sich der Indikator auf den Netto-Inspirations-Volumenfluss oder auf die Fließrichtung bezieht. Falls man diese beiden Informationen hingegen mit einem einzigen Indikator darstellen würde, so hätte dieser Indikator 3 * 3 = 9 verschiedene mögliche Werte und lässt sich nicht so schnell und / oder nicht so zuverlässig erfassen.

Erfindungsgemäß zeigt ein Anzeigeelement die Größe (den Betrag) und die Richtung eines Volumenflusses an. Nicht erforderlich ist es, vorab festzulegen, wann diese Größe oder Richtung korrekt ist und wann sie falsch ist, so dass ein Alarm generiert und ausgegeben werden muss.

Erfindungsgemäß ermittelt das Steuergerät den Netto-Inspirations-Volumenfluss und veranlasst, dass dieser visuell angezeigt wird. Das Steuergerät ermittelt also nicht oder wenigstens nicht nur den gesamten Volumenfluss durch die Inspirations-Fluidführungseinheit hindurch, sondern denjenigen Anteil an dem gesamten Volumenfluss, der durch die Beatmungshübe des Beatmungsgeräts erzeugt wird. Dadurch wird die Gefahr reduziert, dass ein auf andere Weise entstandener Volumenfluss eine ausreichende Aktivität des Beatmungsgeräts vortäuscht, obwohl das Beatmungsgerät überhaupt keine oder zu geringe Beatmungshübe ausführt.

Erfindungsgemäß ermittelt das Steuergerät den Netto-Inspirations-Volumenfluss durch die Inspirations-Fluidführungseinheit hindurch, also den Volumenfluss, den das Beatmungsgerät erzeugt. Dieser Netto-Inspirations-Volumenfluss tritt außerhalb des Beatmungsgeräts auf. Angezeigt wird also nicht ein Volumenfluss innerhalb des Beatmungsgeräts, sondern ein Volumenfluss zwischen dem Beatmungsgerät und der patientenseitigen Koppeleinheit. Dadurch ermöglicht es die Erfindung, nicht nur die Auswirkung eines Fehlers im Beatmungsgerät zu erkennen, sondern auch die Auswirkung eines Fehlers zwischen dem Beatmungsgerät und der patientenseitigen Koppeleinheit.

In einer Ausgestaltung umfasst das Beatmungssystem einen Inspirations-Volumenfluss-Sensor. Dieser Inspirations-Volumenfluss-Sensor vermag ein Maß für den Volumenfluss durch die Inspirations-Fluidführungseinheit hindurch zu messen. Das Steuergerät empfängt und verarbeitet ein Signal des Inspirations-Volumenfluss-Sensors, um das Maß für den Netto-Inspirations-Volumenfluss zu ermitteln. Das empfangene und verarbeitete Signal umfasst eine Information über den Volumenfluss. Möglich ist, dass der Volumenfluss-Sensor automatisch Roh-Messwerte aufbereitet, beispielsweise glättet, und/oder Ausreißer erkennt und ausschließt. Insbesondere aggregiert das Steuergerät über mehrere Signalwerte des Inspirations-Volumenfluss-Sensors für verschiedene Mess-Zeitpunkte. Die Aggregation umfasst insbesondere eine Mittelung oder gewichtete Mittelung über mehrere Messwerte.

In einer Ausgestaltung ermittelt das Steuergerät im Signal des Inspirations-Volumenfluss-Sensors die Inspirationsphasen und / oder einen oszillierenden Anteil. Zumindest bei fehlerfrei arbeitendem Atmungssystem fließt in jeder Inspirationsphase jeweils eine ausgestoßene Menge des Gasgemischs vom Beatmungsgerät zur patientenseitigen Koppeleinheit. Wenigstens in jeder Inspirationsphase wird der oszillierende Anteil vom Beatmungsgerät erzeugt. Das Steuergerät ermittelt die Frequenz und / oder Amplitude des oszillierenden Anteils.

In einer Ausgestaltung erfasst das Steuergerät von einer übergeordneten Steuerung eine Soll-Frequenz und / oder eine Soll-Amplitude der Beatmungshübe. Durch diesen Vergleich stellt das Steuergerät fest, ob der oszillierende Anteil tatsächlich vom Beatmungsgerät stammt. Optional verwendet das Steuergerät hierfür zusätzlich die Kenntnis der Inspirationsphasen.

Die Ausgestaltung mit dem Volumenfluss-Sensor ermöglicht es, den Volumenfluss durch die Inspiration-Fluidführungseinheit hindurch direkt an der Inspirations-Fluidführungseinheit zu ermitteln und nicht an einer räumlich entfernten Messstelle. Eine Messung an einer räumlich entfernten Messstelle kann zu größeren Fehlern führen als eine Messung direkt an der Inspirations-Fluidführungseinheit.

Die Inspirations-Fluidführungseinheit verbindet die patientenseitige Koppeleinheit mit dem Beatmungsgerät. In einer Ausgestaltung ist kein Beatmungskreislauf hergestellt, d.h. die vom Patienten ausgeatmeten Luft gelangt in die Umgebung. Insbesondere dann, wenn kein Beatmungskreislauf hergestellt ist, ist das Signal des Inspirations-Volumenfluss-Sensors eine gute Näherung für den zu ermittelnden Netto-Inspirations-Volumenfluss. In einer Ausgestaltung verwendet das Steuergerät den vom Inspirations-Volumenfluss-Sensor gemessenen Volumenfluss als den Netto-Inspirations-Volumenfluss. In einer anderen Ausgestaltung verwendet das Steuergerät Signale von weiteren Sensoren, die jeweils ein Maß für weitere Volumenflüsse messen.

Erfindungsgemäß ermittelt das Steuergerät ein Maß für den Netto-Inspirations-Volumenfluss. Gemäß der gerade beschriebenen bevorzugten Ausgestaltung verwendet das Steuergerät hierfür ein Signal des Inspirations-Volumenfluss-Sensors. In einer Realisierungsform leitet das Steuergerät aus dem Signal, bevorzugt aus einem Signalwert, einen Wert für den aktuellen Netto-Inspirations-Volumenfluss her und verwendet diesen für die Ansteuerung des Inspirations-Anzeigeelements. In einer anderen Realisierungsform mittelt das Steuergerät über mehrere Signalwerte vom Inspirations-Volumenfluss-Sensor oder aggregiert auf andere Weise, z.B. als ein Median, mehrere Werte und leitet bevorzugt einen gemittelten Netto-Inspirations-Volumenfluss her. Diese Realisierungsform führt in vielen Fällen dazu, dass der Indikator für den Volumenfluss, den das Anzeigeelement anzeigt, weniger stark von Schwankungen des Netto-Inspirations-Volumenflusses abhängt.

Erfindungsgemäß ist das Inspirations-Anzeigeelement an der Inspirations-Fluidführungseinheit oder an einer weiteren Fluidführungseinheit befestigt, wobei die weitere Fluidführungseinheit in einer Fluidverbindung mit dem Beatmungsgerät und mit der Inspirations-Fluidführungseinheit steht. In einer Ausgestaltung ist diese weitere Fluidführungseinheit ein Inspirations-Anschlussstück, wobei dieses Anschlussstück insbesondere als eine Röhre ausgestaltet ist. Das Inspirations-Anschlussstück ist ein Teil des Beatmungsgeräts und bevorzugt außen an einem Gehäuse des Beatmungsgeräts befestigbar oder befestigt, besonders bevorzugt dauerhaft befestigt. Die Inspirations-Fluidführungseinheit lässt sich lösbar und fluiddicht mit dem Inspirations-Anschlussstück verbinden. Beispielsweise wird ein Schlauch der Inspirations-Fluidführungseinheit über die Röhre geschoben. Selbstverständlich ist es möglich, dass aufgrund eines Fehlers die Inspirations-Fluidführungseinheit überhaupt nicht oder nicht fluiddicht mit dem Inspirations-Anschlussstück verbunden ist oder dass ein Filter vor oder nach einer Fluidführungseinheit verstopft ist, was in vielen Fällen ein Benutzer dank der Erfindung bemerken kann. Wenn die Inspirations-Fluidführungseinheit mit dem Inspirations-Anschlussstück verbunden ist, ist eine Fluidverbindung zwischen der Inspirations-Fluidführungseinheit und dem Beatmungsgerät hergestellt.

Diese Ausgestaltung ermöglicht es, das Inspirations-Anzeigeelement am Beatmungsgerät zu befestigen. Es ist dann kein Teil der Inspirations-Fluidführungseinheit und kann daher nicht auf die Inspirations-Fluidführungseinheit einwirken. Falls die Inspirations-Fluidführungseinheit vom Inspirations-Anschlussstück getrennt wird, bleibt das Inspirations-Anzeigeelement am Beatmungsgerät vorhanden. Obwohl das Inspirations-Anzeigeelement nicht zur Inspirations-Fluidführungseinheit gehört, ist es in der Nähe der Inspirations-Fluidführungseinheit angeordnet. Es vermag anzuzeigen, ob und wenn ja in welche Richtung ein Fluid durch das Inspirations-Anschlussstück und damit durch die Inspirations-Fluidführungseinheit fließt.

In einer Ausgestaltung realisiert das Beatmungssystem einen Beatmungskreislauf. Diese Ausgestaltung wird bevorzugt insbesondere dann angewendet, wenn der Patient narkotisiert ist, die von ihm ausgeatmete Luft daher mindestens ein Narkosemittel umfasst und dieses Narkosemittel nicht in die Umgebung gelangen soll. Eine Exspirations-Fluidführungseinheit des Beatmungssystems leitet ein Gasgemisch, welches aus der patientenseitigen Koppeleinheit austritt, in der Regel die vom Patienten ausgeatmeten Luft, zum Beatmungsgerät. Ein Exspirations-Anzeigeelement ist an der Exspirations-Fluidführungseinheit oder an einer anderen Fluidführungseinheit befestigt, wobei diese andere Fluidführungseinheit in einer Fluidverbindung mit dem Beatmungsgerät und mit der Exspirations-Fluidführungseinheit steht.

Das Steuergerät vermag gemäß dieser Ausgestaltung ein Maß für den Exspirations-Volumenfluss zu ermitteln. Dieser Exspirations-Volumenfluss ist der Volumenfluss, der von der patientenseitigen Koppeleinheit durch die Exspirations-Fluidführungseinheit hindurch zum Beatmungsgerät fließt. Das Steuergerät vermag das Exspirations-Anzeigeelement anzusteuern, und zwar abhängig vom ermittelten Exspirations-Volumenfluss. Das Exspirations-Anzeigeelement vermag zwei Indikatoren anzuzeigen, und zwar abhängig von der Ansteuerung durch das Steuergerät und in einer von einem Menschen visuell wahrnehmbaren Weise, nämlich die folgenden beiden Indikatoren:

- einen Indikator für die Größe (den Betrag) des ermittelten Exspirations-Volumenflusses und

- einen Indikator für die Fließrichtung des Gasgemischs, welches durch die Exspirations-Fluidführungseinheit hindurchfließt.

In einer einfachen Ausgestaltung zeigt das Exspirations-Anzeigeelement an, ob überhaupt ein Fluid mit einem Volumenfluss oberhalb einer vorgegebenen unteren Schranke durch die Exspirations-Fluidführungseinheit fließt oder nicht.

Die Ausgestaltung mit zwei verschiedenen Anzeigeelementen, die voneinander räumlich beabstandet sind, erleichtert es einem Benutzer weiter, einen Fehler zu erkennen und zu lokalisieren. Ein Fehler kann insbesondere in Verbindung zwischen dem Beatmungsgerät einerseits und der Inspirations-Fluidführungseinheit und / oder mit der Exspirations-Fluidführungseinheit andererseits auftreten.

Weiter oben wurde eine Realisierungsform beschrieben, bei der das Inspirations-Anzeigeelement an einem Inspirations-Anschlussstück des Beatmungsgeräts befestigt ist, wobei die Inspirations-Fluidführungseinheit sich fluiddicht und lösbar mit dem Inspirations-Anschlussstück verbinden lässt. Eine entsprechende Realisierungsform ist auch für das Exspirations-Anzeigeelement möglich. Die Exspirations-Fluidführungseinheit lässt sich lösbar und fluiddicht mit einem Exspirations-Anschlussstück verbinden, wobei das Exspirations-Anschlussstück bevorzugt als eine Röhre ausgestaltet ist, die außen an einem Gehäuse des Beatmungsgeräts befestigt ist. Die Vorteile, die weiter oben mit Bezug auf das Inspirations-Anschlussstück beschrieben wurden, lassen sich entsprechend auch für das Exspirations-Anschlussstück erzielen.

Falls ein Beatmungskreislauf hergestellt ist, speist das Beatmungsgerät ein Gasgemisch, welches durch die Exspirations-Fluidführungseinheit hindurch zum Beatmungsgerät fließt, wieder in die Inspirations-Fluidführungseinheit ein. Bevorzugt entzieht das Beatmungsgerät zuvor diesem Gasgemisch Kohlendioxid. Der Volumenfluss durch die Inspirations-Fluidführungseinheit umfasst daher eine Überlagerung des Netto-Inspirations-Volumenflusses, den das Beatmungsgerät erzeugt, und des Volumenflusses durch die Exspirations-Fluidführungseinheit hindurch, abzüglich dem Volumenfluss des entzogenen Kohlendioxids.

In einer bevorzugten Ausgestaltung umfasst das Beatmungssystem daher einen Inspirations-Volumenfluss-Sensor und einen Exspirations-Volumenfluss-Sensor. Der Inspirations-Volumenfluss-Sensor misst ein Maß für den gesamten Volumenfluss durch die Inspirations-Fluidführungseinheit, der Exspirations-Volumenfluss-Sensor ein Maß für den Volumenfluss durch die Exspirations-Fluidführungseinheit. Der optionale Inspirations-Volumenfluss-Sensor misst in der Regel ein Maß für dem Volumenfluss, der durch die oben erwähnte Überlagerung entsteht, wobei optional mindestens ein weiterer Volumenfluss hinzukommt. Das Steuergerät ermittelt den Netto-Inspirations-Volumenfluss abhängig von jeweils einem Signal des Inspirations-Volumenfluss-Sensors und des Exspirations-Volumenfluss-Sensors. Bei der Ausgestaltung, bei der ein Beatmungskreislauf hergestellt ist, umfasst das Beatmungssystem das erfindungsgemäße Inspirations-Anzeigeelement sowie das bevorzugte oben beschriebene Exspirations-Anzeigeelement. Die Merkmale, dass das Beatmungssystem gemäß dieser Ausgestaltung zwei räumlich voneinander getrennte Anzeigeelemente umfasst und das Inspirations-Anzeigeelement abhängig vom Netto-Inspirations-Volumenfluss angesteuert wird, erleichtert es einem Benutzer, einen Fehler zu lokalisieren. Insbesondere wird es einem Benutzer erleichtert, einen Fehler an der Inspirations-Fluidführungseinheit von einem Fehler an der Exspirations-Fluidführungseinheit zu unterscheiden.

Die weiter oben und auch nachfolgend beschriebenen vorteilhaften Ausgestaltungen und Realisierungsformen des Inspirations-Anzeigeelements sind entsprechend auch vorteilhafte Ausgestaltungen und Realisierungsformen des Exspirations-Anzeigeelements.

Erfindungsgemäß zeigt das Inspirations-Anzeigeelement einen Indikator für den Netto-Inspirations-Volumenfluss durch die Inspirations-Fluidführungseinheit an. Im einfachsten Falle zeigt dieser Indikator an, ob ein Volumenfluss oberhalb einer vorgegebenen unteren Schranke durch die Inspirations-Fluidführungseinheit fließt oder nicht. In einer anderen Realisierungsform zeigt dieser Indikator zusätzlich ein kontinuierliches oder gestuftes Maß für die Größe des Volumenflusses an. Dieses Maß für die Größe des Volumenflusses hängt vom Netto-Inspirations-Volumenfluss ab, den das Steuergerät so wie oben beschrieben ermittelt.

In einer Realisierungsform ist dieses Maß für die Größe des Volumenflusses für jeden Patienten das gleiche und hängt nur vom Netto-Inspirations-Volumenfluss ab. In einer anderen Realisierungsform wird hingegen berücksichtigt, dass sich auch bei einem fehlerfreien Betrieb der Netto-Inspirations-Volumenfluss von Patient zu Patient stark unterscheiden kann. Insbesondere ist der Netto-Inspirations-Volumenfluss bei einem Kind in der Regel wesentlich kleiner als bei einem Erwachsenen. Der Netto-Inspirations-Volumenfluss kann auch vom Zustand der Lunge des Patienten und / oder von einem Vorgehen bei der künstlichen Beatmung abhängen. Die folgende Realisierungsform erleichtert es einem Benutzer, trotz dieser Unterschiede einen Fehler an der Inspirations-Fluidführungseinheit festzustellen.

Gemäß dieser Realisierungsform vermag das Steuergerät ein Maß für einen maximalen Soll-Volumenfluss zu erfassen. Dieser maximale Soll-Volumenfluss ist vorgegeben und soll vom Beatmungsgerät durch das Ausstoßen des Gasgemischs in den Beatmungshüben erzeugt werden. Der maximale Soll-Volumenfluss kann insbesondere von einem Benutzer oder von einer übergeordneten Steuerung vorgegeben werden. Beispielsweise wird der maximale Soll-Volumenfluss abhängig von der Größe, dem Alter und / oder dem Gewicht des Patienten hergeleitet. Der maximale Soll-Volumenfluss kann als ein zeitlicher Verlauf vorgegeben werden, insbesondere als ein oszillierender Verlauf, oder als ein Maximalwert oder Mittelwert. Dieser maximale Soll-Volumenfluss variiert in der Regel von Patient zu Patient. Idealerweise liegt der Netto-Inspirations-Volumenfluss durch die Inspirations-Fluidführungseinheit hindurch in einem vorgegebenen Toleranzband unter diesem maximalen Soll-Volumenfluss. Das Steuergerät vermag einen Quotienten zu berechnen, wobei der ermittelte (tatsächliche) Netto-Inspirations-Volumenfluss im Zähler und der erfasste maximale Soll-Volumenfluss im Nenner dieses Quotienten auftritt. Das Steuergerät vermag das Inspirations-Anzeigeelement abhängig von den berechneten Quotienten anzusteuern. Der Indikator für die Größe des Volumenflusses hängt von dem berechneten Quotienten ab. Diese Ausgestaltung erspart es einem Benutzer, eine Anzeige auf dem Anzeigeelement mit einem zu erzielenden maximalen Volumenfluss zu vergleichen und zu bewerten.

Eine Weiterbildung dieser Ausgestaltung lässt sich insbesondere dann anwenden, wenn ein Beatmungskreislauf hergestellt ist und eine Exspirations-Fluidführungseinheit von der patientenseitigen Koppeleinheit zurück zum Beatmungsgerät führt. Ein Exspirations-Volumenfluss-Sensor vermag ein Maß für den Volumenfluss durch die Exspirations-Fluidführungseinheit hindurch zu messen. Auch der Volumenfluss durch die Exspirations-Fluidführungseinheit soll in der Regel in einem Toleranzband unterhalb des maximalen Soll-Volumenflusses liegen. Das Steuergerät vermag als weiteren Quotienten den Quotienten aus dem ermittelten Volumenfluss durch die Exspirations-Fluidführungseinheit hindurch und dem maximalen Soll-Volumenfluss zu berechnen und das Exspirations-Anzeigeelement abhängig von diesem weiteren Quotienten anzusteuern.

Erfindungsgemäß vermag das Steuergerät das Inspirations-Anzeigeelement anzusteuern. Das angesteuerte Inspirations-Anzeigeelement zeigt zwei Indikatoren an. Ein Indikator ist ein Maß für die Größe (den Betrag) des Netto-Inspirations-Volumenflusses durch die Inspirations-Fluidführungseinheit hindurch. Unterschiedliche Realisierungsformen sind möglich, wie das Inspirations-Anzeigeelement diesen Indikator anzeigt.

Bevorzugt sind die beiden Indikatoren so angeordnet, dass ein Benutzer auf einen Blick beide Indikatoren wahrnehmen kann. Beispielsweise sind die beiden Indikatoren übereinandergelegt oder benachbart zueinander angeordnet. Nicht erforderlich ist, dass ein Benutzer den Blick abwechselnd auf den einen und auf den anderen Indikator richten muss.

In einer Ausgestaltung nimmt der Indikator für den Netto-Inspirations-Volumenfluss einen von mehreren möglichen Farbwerten oder eine Darstellung von mehreren möglichen sonstigen visuell wahrnehmbaren Darstellungen an, wobei jeder Farbwert bzw. jede sonstige wahrnehmbare Darstellung mit jeweils einem Wertebereich des Netto-Inspirations-Volumenflusses verknüpft ist. Der Indikator für die Fließrichtung ist in einer Ausgestaltung entweder einer von zwei möglichen Pfeilen oder ein sonstiges Symbol, wobei die beiden Pfeile einander entgegengesetzt sind und die Fließrichtung anzeigen wobei das sonstige Symbol anzeigt, dass der Volumenfluss unter einer vorgegebenen unteren Schranke liegt.

In einer ersten Realisierungsform vermag das Steuergerät durch die Ansteuerung zu bewirken, dass das Inspirations-Anzeigeelement mit einer veränderlichen Helligkeit leuchtet. Bevorzugt ist die Helligkeit umso größer, je größer die darzustellende Größe des Netto-Inspirations-Volumenflusses ist. Bevorzugt zeigt das nicht leuchtende Inspirations-Anzeigeelement an, dass kein Fluid mit einem Volumenfluss oberhalb einer unteren Volumenfluss-Schranke durch die Inspirations-Fluidführungseinheit hindurchfließt. Möglich ist, dass das Inspirations-Anzeigeelement mehrere mögliche Helligkeitsstufen aufweist. Möglich ist auch, dass das Steuergerät durch eine entsprechende Ansteuerung die Helligkeit kontinuierlich zu verändern vermag.

In einer Realisierungsform ist die jeweilige maximale Helligkeit jedes Anzeigeelements fest vorgegeben und lässt sich nicht verändern. In einer anderen Ausgestaltung ist die maximale Helligkeit veränderbar. Vorgegeben ist ein Standardwert für die maximale Helligkeit. Dieser Standardwert wird beispielsweise dann erreicht, wenn der ermittelte Volumenfluss bis auf eine Toleranz gleich dem oben beschriebenen maximalen Soll-Volumenfluss ist. Ein Benutzer kann diesen Standardwert verändern. Möglich ist auch, dass ein Helligkeits-Sensor die Helligkeit in der Umgebung des Beatmungssystems misst und das Steuergerät die jeweilige maximale Helligkeit eines Anzeigeelements abhängig von der gemessenen Helligkeit in der Umgebung verändert, nämlich dergestalt, dass die maximale Helligkeit des Anzeigeelements umso größer ist, je größer die Umgebungs-Helligkeit ist. Die Ausgestaltung mit der veränderbaren maximalen Helligkeit erleichtert es, das Anzeigeelement an Umgebungsbedingungen anzupassen, und reduziert die Gefahr, dass ein Fehler wegen einer zu geringen Helligkeit des Anzeigeelements übersehen wird oder dass umgekehrt das Anzeigeelement als zu hell empfunden wird oder eine andere Meldeleuchte visuell überdeckt oder überstrahlt.

In einer zweiten Realisierungsform vermag das Steuergerät durch die Ansteuerung zu bewirken, dass das Inspirations-Anzeigeelement mit einer veränderlichen Frequenz blinkt und / oder flackert. Diese Frequenz ist ein Indikator für die Größe des Netto-Inspirations-Volumenflusses. Bevorzugt ist die Frequenz umso größer, je größer der Netto-Inspirations-Volumenfluss ist.

Die beiden gerade beschriebenen Realisierungsformen lassen sich miteinander kombinieren. Möglich ist auch, dass zusätzlich oder stattdessen eine Farbe als Indikator für die Größe des Netto-Inspirations-Volumenflusses verwendet wird, beispielsweise eine der drei Farben grün, gelb, rot (Ampelfunktion). Bevorzugt bedeutet grün einen Volumenfluss innerhalb eines Soll-Toleranzbands, gelb einen Volumenfluss außerhalb des Soll-Toleranzbands, aber noch innerhalb eines breiteren Toleranzbands, und rot einen Volumenfluss außerhalb des breiteren Toleranzbands.

In einer Ausgestaltung umfasst das Beatmungssystem zusätzlich einen Gasmischer. Dieser Gasmischer vermag aus mindestens zwei zugeführten Gas-Bestandteilen ein Gasgemisch zu erzeugen. Mindestens ein Gas-Bestandteil ist oder umfasst Sauerstoff. Möglich ist, dass mindestens ein weiterer Gas-Bestandteil ein Narkosemittel ist oder umfasst. Der Gasmischer steht wenigstens zeitweise in einer Fluidverbindung mit dem Beatmungsgerät, und das erzeugte Gasgemisch wird zu dem und in das Beatmungsgerät gefördert. Die Gas-Bestandteile werden von jeweils einem stationären oder mobilen Versorgungsanschluss bereitgestellt.

In einer Fortbildung dieser Ausgestaltung ist an einer Fluidführungseinheit ein Gasmischer-Anzeigeelement angebracht. Die Fluidführungseinheit befindet sich an einem Ausgang des Gasmischers Das Steuergerät vermag dieses Gasmischer-Anzeigeelement anzusteuern. Das angesteuerte Gasmischer-Anzeigeelement zeigt mindestens einen Indikator an, nämlich einen Indikator für die Größe des Volumenflusses durch den Ausgang aus dem Gasmischer heraus.

In einer Ausgestaltung umfasst das Beatmungssystem zusätzlich eine Bypass-Fluidführungseinheit und einen pneumatischen Schalter. Die Bypass-Fluidführungseinheit umgeht das Beatmungsgerät und steht in einer Fluidverbindung mit der patientenseitigen Koppeleinheit. Der Schalter vermag ein Gasgemisch wahlweise in das Beatmungsgerät oder in die Bypass-Fluidführungseinheit zu leiten. **In** einer Ausgestaltung steht ein Eingang des Schalters in einer Fluidverbindung mit dem gerade erwähnten Gasmischer. Diese Ausgestaltung erhöht die Flexibilität des Beatmungssystems. Das Beatmungssystem lässt sich wahlweise

- für eine künstliche Beatmung, bei welcher der Patient durch das Beatmungsgerät beatmet wird und optional vollständig narkotisiert ist, oder
- für eine Beatmung, bei der der Patient mit seiner eigenen Atmungsaktivität ein Gasgemisch ansaugt und einatmet,

verwenden. Dieses Gasgemisch stammt beispielsweise von dem gerade erwähnten Gasmischer.

Gemäß der gerade erwähnten Ausgestaltung ist an der Bypass-Fluidführungseinheit oder an einer weiteren Fluidführungseinheit, die in einer Fluidverbindung mit der Bypass-Fluidführungseinheit steht, ein Bypass-Anzeigeelement angebracht. Das Steuergerät vermag dieses Bypass-Anzeigeelement anzusteuern. Das Bypass-Anzeigeelement vermag mindestens einen Indikator in einer visuell wahrnehmbaren Weise anzuzeigen, nämlich einen Indikator für die Größe des Volumenflusses durch die Bypass-Fluidführungseinheit hindurch. Optional vermag das Bypass-Anzeigeelement zusätzlich einen Indikator für die Richtung, in die ein Fluid durch die Bypass-Fluidführungseinheit hindurchfließt, anzuzeigen. In vielen Fällen ist an der Bypass-Fluidführungseinheit dieser Indikator für die Fließrichtung hingegen nicht erforderlich, weil oft kein Fluid von der patientenseitigen Koppeleinheit in die Bypass-Fluidführungseinheit hineinfließt. Optional misst ein Volumenfluss-Sensor ein Maß für den Volumenfluss durch die Bypass-Fluidführungseinheit hindurch. In einer Ausgestaltung misst dieser Sensor ein Maß für den Volumenfluss aus dem gerade erwähnten Gasmischer.

Die Ausgestaltung mit dem Bypass-Anzeigeelement erleichtert es einem Benutzer weiter, einen möglichen Fehler zu finden, und zwar sowohl dann, wenn der Patient vom Beatmungsgerät versorgt wird, als auch dann, wenn der Patient ein Gasgemisch aus der Bypass-Fluidführungseinheit erhält und einatmet.

Erfindungsgemäß steuert das Steuergerät das Inspirations-Anzeigeelement an, und das angesteuerte Inspirations-Anzeigeelement zeigt zwei Indikatoren an, nämlich einen Indikator für die Größe (den Betrag) des ermittelten Netto-Inspirations-Volumenflusses und einen Indikator für die Fließrichtung des Gasgemischs. In einer Ausgestaltung erfasst das Steuergerät zusätzlich ein Maß für einen geforderten Netto-Inspirations-Volumenfluss. Dieses Maß umfasst mindestens die Information, dass jetzt ein Gasgemisch aus dem Beatmungsgerät heraus in die Inspirations-Fluidführungseinheit hinein fließen soll. In einer Realisierungsform erfasst das Steuergerät diesen geforderten Netto-Inspirations-Volumenfluss, indem das Steuergerät automatisch eine Anfrage an eine übergeordnete Steuerung oder Regelung des Beatmungssystems richtet und eine Antwort verarbeitet. Durch eine Ansteuerung bewirkt das Steuergerät, dass das angesteuerte Inspirations-Anzeigeelement mindestens einen der beiden Indikatoren zusätzlich in Abhängigkeit von dem erfassten geforderten Netto-Inspirations-Volumenfluss ansteuert. Insbesondere wird mindestens ein Indikator hervorgehoben dargestellt, wenn das ermittelten Maß für den tatsächlichen Volumenfluss erheblich, also um mehr als eine vorgegebene Toleranz, von dem erfassten geforderten Volumenfluss abweicht. Dies ist insbesondere dann der Fall, wenn das Steuergerät erfasst hat, dass ein Gasgemisch aus dem Beatmungsgerät heraus in die Inspirations-Fluidführungseinheit fließen soll, der tatsächlich ermittelte Volumenfluss aber unterhalb einer vorgegebenen unteren Schranke liegt.

Diese Ausgestaltung erleichtert es einem Benutzer weiter, rasch einen Hinweis auf einen Fehler festzustellen.

Im Folgenden wird die Erfindung anhand eines Ausführungsbeispiels beschrieben. Hierbei zeigt

Figur 1    schematisch das Beatmungsgerät, den Gasmischer und die Verbindung mit dem Patienten, wobei die künstliche Beatmung die eigene Atmungsaktivität des Patienten ergänzt;

Figur 2    schematisch eine Abwandlung der Ausgestaltung von Figur 1, wobei der Patient narkotisiert ist und wobei ein Beatmungskreislauf hergestellt ist;

Figur 3    die Anzeigeelemente für die Inspirationsleitung und die Exspirationsleitung;

Figur 4    mittels eines Flussdiagramms, wie die Ansteuerung des Inspirations-Anzeigeelements hergeleitet wird;

Figur 5    das Anzeigeelement für die Bypassleitung.

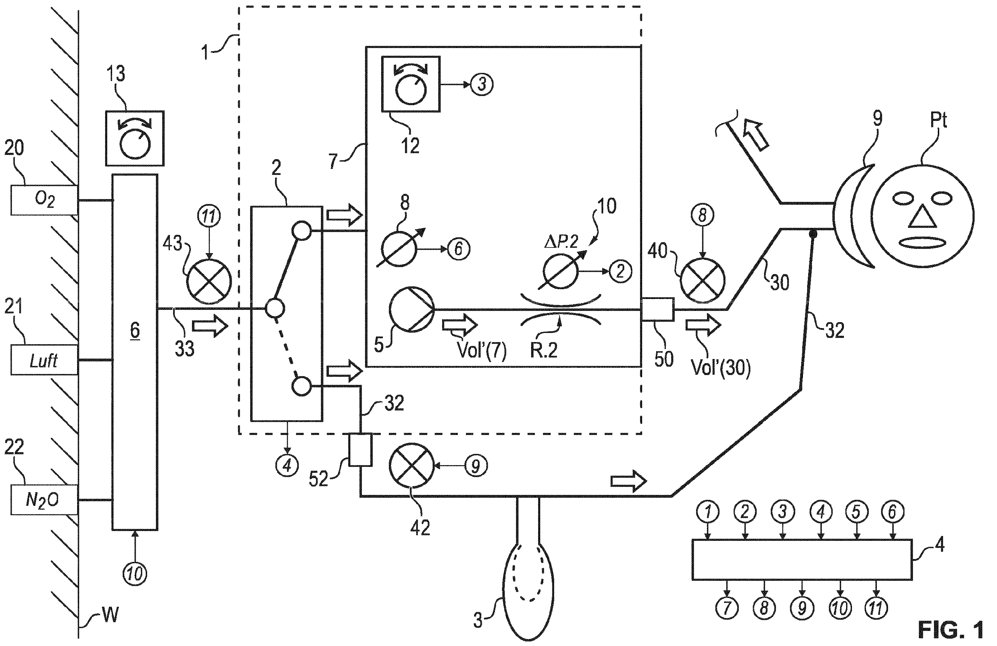

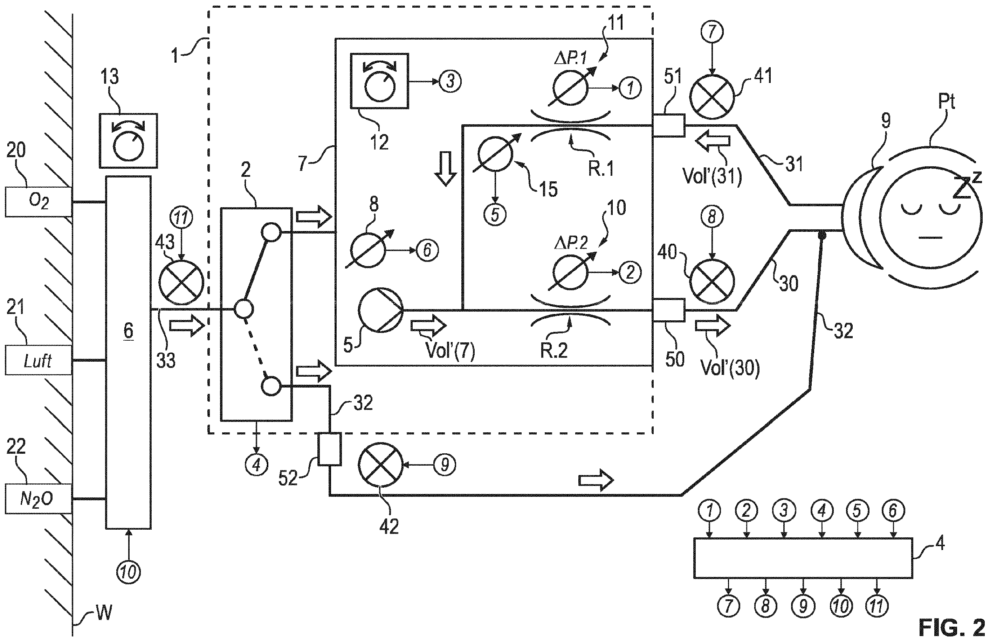

Figur 1 und Figur 2 zeigen schematisch eine Verwendung der Erfindung für die künstliche Beatmung eines Patienten Pt. Gleiche Bezugszeichen haben in Figur 1 und Figur 2 die gleiche Bedeutung. Auf oder an oder im Körper des Patienten Pt ist eine patientenseitige Koppeleinheit 9 angeordnet. Im gezeigten Beispiel ist eine Atemmaske 9 auf das Gesicht des Patienten Pt gelegt. Im Beispiel von Figur 1 entwickelt der Patient Pt eine eigene Atmungsaktivität, die von der Atmungsmuskulatur des Patienten Pt ausgeführt wird. Diese eigene Atmungsaktivität kann eine spontane Atmung sein und / oder extern stimuliert werden, beispielsweise in einem elektromagnetischen Feld. Eine Vorrichtung, die in einem solchen Feld die Atmungsmuskulatur eines Patienten aktiviert, wird beispielsweise in WO 2019 / 154834 A1 beschrieben. Im Beispiel von Figur 2 ist der Patient Pt hingegen narkotisiert oder wenigstens sediert.

Der patientenseitigen Koppeleinheit 9 und damit dem Patienten Pt wird ein Inspirations-Gasgemisch über eine Inspirationsleitung 30 zugeführt. Das Inspirations-Gasgemisch umfasst Sauerstoff und in der gezeigten Anwendung von Figur 2 zusätzlich mindestens ein Narkosemittel. Das vom Patienten Pt ausgeatmete Exspirations-Gasgemisch wird in der Ausgestaltung gemäß Figur 2 von der patientenseitigen Koppeleinheit 9 über eine Exspirationsleitung 31 abgeführt. Die jeweilige Fließrichtung des Gasgemischs wird in Figur 1 und Figur 2 durch Pfeile angedeutet. Bevorzugt ist zwischen der patientenseitigen Koppeleinheit 9 und den beiden Leitungen 30 und 31 ein Y-Stück angeordnet. Möglich ist auch, dass ein Zwei-Lumen-Schlauch sowohl die Inspirationsleitung 30 als auch die Exspirationsleitung 31 realisiert.

Die Inspirationsleitung 30 ist an einem Inspirations-Anschlussstück 50 befestigt, die Exspirationsleitung 31 an einem Exspirations-Anschlussstück 51. Die beiden Anschlussstücke 50, 51 sind außen an einem Gehäuse eines schematisch dargestellten Beatmungsgeräts (Ventilators) 1 befestigt. In der Anwendung gemäß Figur 1 fließt die vom Patienten Pt ausgeatmete Luft in die Umgebung.

Möglich ist, dass eine Leitung 30, 31 an einem falschen Anschlussstück 51, 50 oder fälschlicherweise überhaupt nicht an einem Anschlussstück befestigt ist.

Das Beatmungsgerät 1 umfasst eine Antriebseinheit 7, bevorzugt mit einer Pumpe und / oder einem Gebläse 5.

Ein nur schematisch gezeigtes signalverarbeitendes Steuergerät (control unit) 4 steuert die Antriebseinheit 7 an. Die Antriebseinheit 7 bewirkt, dass das Beatmungsgerät 1 eine Abfolge von Beatmungshüben durchführt. In jedem Beatmungshub stößt das Beatmungsgerät 1 jeweils eine Menge des Inspirations-Gasgemischs aus. Die ausgestoßene Menge wird durch das Inspirations-Anschlussstück 50 hindurch in die Inspirationsleitung 30 hinein und durch die Inspirationsleitung 30 hindurch zur patientenseitigen Koppeleinheit 9 gefördert. Das Exspirations-Gasgemisch fließt im Beispiel von Figur 2 von der patientenseitigen Koppeleinheit 9 durch die Exspirationsleitung 31 und durch das Exspirations-Anschlussstück 51 hindurch wieder zum Beatmungsgerät 1.

Möglich ist, dass die Antriebseinheit 7 nicht so wie gewünscht arbeitet. Dies kann zur Folge haben, dass das Inspirations-Gasgemisch nicht oder nicht mit ausreichendem Volumenfluss durch die Inspirationsleitung 30 hindurchfließt, sondern z.B. wenigstens ein Teil im Beatmungsgerät 1 verbleibt.

Das Beatmungsgerät 1 unterstützt und ergänzt in der Ausgestaltung gemäß Figur 1 die eigene Atmungsaktivität des Patienten Pt, und das Exspirations-Gasgemisch wird in die Umgebung geleitet. In einer anderen Ausgestaltung, die in Figur 2 gezeigt wird, realisiert das Beatmungsgerät 1 einen Beatmungskreislauf, bei dem das Beatmungsgerät 1 dem Exspirations-Gasgemisch Kohlendioxid ($CO_2$) entzieht und das verbleibende Gasgemisch wieder in die Inspirationsleitung 30 einspeist. In einer Ausgestaltung misst ein optionaler Kohlendioxid-Sensor 15 ein Maß für die Konzentration (den Anteil) $CO_2$ von Kohlendioxid im Exspirations-Gasgemisch, das durch die Exspirationsleitung 31 fließt.

Ein Volumenfluss-Sensor 10 misst ein Maß für den Volumenfluss Vol'(30) durch die Inspirationsleitung 30. Der Volumenfluss ist das Volumen pro Zeiteinheit eines Fluids, welches durch die Inspirationsleitung 30 fließt. Falls ein Beatmungskreislauf realisiert ist, so misst der Volumenfluss-Sensor 10 einen Volumenfluss, der eine Überlagerung des Volumenflusses, der durch die Beatmungshübe der Antriebseinheit 7 erzeugt wird, und des Volumenflusses, der durch die Einspeisung des ausgeatmeten Gasgemischs (Exspirations-Gasgemischs) erzeugt wird, umfasst, abzüglich des Volumenflusses des herausgefilterten Kohlendioxids. Ein Volumenfluss-Sensor 11 misst ein Maß für den Volumenfluss Vol'(31) durch die Exspirationsleitung 31.

Nachfolgend wird beispielhaft eine Realisierungsform des Volumenfluss-Sensors 10 beschrieben. Andere Realisierungsformen sind ebenfalls möglich. Der Volumenfluss-Sensor 11 kann genauso realisiert sein.

In der Inspirationsleitung 30 ist ein pneumatischer Widerstand R.2 angeordnet. Der Volumenfluss-Sensor 10 misst ein Maß für den Druckunterschied ΔP.2 zwischen dem Druck flussaufwärts und dem Druck flussabwärts vom pneumatischen Widerstand R.2. Entsprechend misst der Volumenfluss-Sensor 11 ein Maß für den Volumenfluss Vol'(31) durch die Exspirationsleitung 31, nämlich den Druckunterschied ΔP.1 zwischen dem Druck flussaufwärts und dem Druck flussabwärts von einem pneumatischen Widerstand R.1 in der Exspirationsleitung 31.

In einer abweichenden Ausgestaltung wird der Volumenfluss Vol'(30) durch die Inspirationsleitung 30 nicht durch einen Volumenfluss-Sensor an der Inspirationsleitung 30 gemessen, sondern durch einen Volumenfluss-Sensor im Beatmungsgerät 1. Alternativ wird der Volumenfluss Vol'(30) durch die Inspirationsleitung 30 aus einem anderen Signal hergeleitet, welches ein Maß für den Volumenfluss ist, den die Antriebseinheit 7 erzeugt. Beispielsweise misst ein Antriebseinheits-Sensor 8 ein Signal, welches ein Maß für den Weg oder den Drehwinkel ist, den eine Fluidfördereinheit der Antriebseinheit 7, beispielsweise die Pumpe 5, in einer Zeiteinheit zurücklegt, oder ein Maß für den Volumenfluss an einem Ausgang der Antriebseinheit 7. Möglich ist auch, dass sowohl der Volumenfluss-Sensor 11 an der Exspirationsleitung 31 als auch der Antriebseinheits-Sensor 8 an der Antriebseinheit 7 jeweils ein Maß für den Volumenfluss messen. In einer Ausgestaltung wird hierdurch Redundanz erzielt.

Ein Gasmischer 6 erzeugt das Inspirations-Gasgemisch aus verschiedenen Gas-Bestandteilen. Mit Hilfe einer Eingabeeinheit 12 am Beatmungsgerät 1 kann ein Benutzer einen gewünschten Volumenfluss des Gasgemischs, welches der Gasmischer 6 bereitstellt und welches aus dem Gasmischer 6 heraus und zum Beatmungsgerät 1 fließt, einstellen. Mithilfe einer optionalen Eingabeeinheit 13 am Gasmischer 6 kann der Benutzer die Konzentration mindestens eines Gas-Bestandteils im Inspirations-Gasgemisch festlegen.

Im gezeigten Beispiel ist der Gasmischer 6 mit einem Versorgungsanschluss 20 für reinen Sauerstoff, einem Versorgungsanschluss 21 für Atemluft und einem Versorgungsanschluss 22 für Lachgas (N2O) oder für ein anderes Narkosemittel verbunden. Diese Anzahl und diese Gas-Bestandteile sind nur beispielhaft zu verstehen. Die drei Versorgungsanschlüsse 20, 21, 22 sind im gezeigten Beispiel stationär in einer Wand W angeordnet. Möglich ist auch, dass die Gas-Bestandteile aus Behältern kommen, insbesondere aus Gasflaschen, die bevorzugt unter Überdruck stehen. Optional umfasst das Beatmungsgerät 1 jeweils eine Halterung für jede Gasflasche.

Die Gas-Bestandteile fließen in den Gasmischer 6 und werden dort zum Inspirations-Gasgemisch vermischt. Der Gasmischer 6 kann einen Narkosemittel-Verdunster oder Narkosemittel-Verdampfer (nicht gezeigt) umfassen, der flüssiges Narkosemittel verdampft und mit einem Trägergas vermischt. Das Trägergas wird beispielsweise aus Gas-Bestandteilen erzeugt, welche die Versorgungsanschlüsse 20, 21, 22 bereitstellen.

Wenn das Inspirations-Gasgemisch ein Narkosemittel enthält, weist auch das Exspirations-Gasgemisch dieses Narkosemittel auf. Um zu verhindern, dass Narkosemittel in die Umgebung gelangt, wird in der Ausgestaltung gemäß Figur 2 ein Beatmungskreislauf realisiert.

Möglich ist auch, dass die Beatmungshübe nicht von der Antriebseinheit 7 des Beatmungsgeräts 1 erzeugt werden, sondern z.B. von einem optionalen Handbeatmungsbeutel 3, den ein Mensch betätigt. Diese Ausgestaltung ist insbesondere dann sinnvoll, wenn das Beatmungsgerät 1 aktuell funktionsunfähig ist. Insbesondere in der Ausgestaltung gemäß Figur 1 ist außerdem möglich, dass der Patient Pt nicht sediert ist und seine eigene Atmungsaktivität ausreicht, um ihn ausreichend mit Atemluft zu versorgen, optional in Verbindung mit einer künstlichen Beatmung, die durch den Handbeatmungsbeutel 3 erzeugt wird. In diesem Falle werden zwar die Versorgungsanschlüsse 20, 21, 22 verwendet, aber nicht die Antriebseinheit 7 des Beatmungsgeräts 1.

Eine Bypassleitung 32 ist mit einem Bypass-Anschlussstück 52 am Beatmungsgerät 1 verbunden, umgeht die Antriebseinheit 7 und die Inspirationsleitung 30 und mündet in die Inspirationsleitung 30 (Figur 1) oder in ein Y-Stück, das mit der patientenseitige Koppeleinheit 9 verbunden ist (Figur 2). In einer Ausgestaltung fungiert das Bypass-Anschlussstück 52 als ein Common Gas Outlet Port. Anstelle der Bypassleitung 32 lässt sich auch eine andere Leitung an das Bypass-Anschlussstück 52 anschließen. In einer Ausgestaltung ist auch in der Bypassleitung 32 ein Volumenfluss-Sensor (nicht gezeigt) angeordnet.

Zum Beatmungsgerät 1 gehört ein pneumatischer Schalter 2, den ein Benutzer betätigen kann. Abhängig von der Stellung dieses Schalters 2 wird das Inspirations-Gasgemisch, welches der Gasmischer 6 erzeugt hat, entweder zur Antriebseinheit 7 oder zum Bypass-Anschlussstück 52 und von dort weiter zur Bypassleitung 32 geleitet. In der in Figur 1 und Figur 2 gezeigten Stellung des Schalters 2 wird das Gasgemisch zur Antriebseinheit 7 geleitet.

Eine Zuführ-Fluidführungseinheit 33 verbindet den Gasmischer 6 mit dem Schalter 2. Ein Gasgemisch, welches der Gasmischer 6 erzeugt hat, fließt durch die Zuführ-Fluidführungseinheit 33 hindurch zum Schalter 2.

Das Steuergerät 4 empfängt jeweils ein Signal von den Volumenfluss-Sensoren 10 und 11, vom Antriebseinheits-Sensor 8, vom $CO_2$-Sensor 15, von den Eingabeeinheiten 12 und 13 und vom Schalter 2, verarbeitet die empfangenen Signale und steuert abhängig vom Ergebnis der Verarbeitung unter anderem den Gasmischer 6 an. Ein Ziel bei dieser Ansteuerung ist, dass das Inspirations-Gasgemisch, welches der Gasmischer 6 bereitstellt, die vom Benutzer vorgegebene Zusammensetzung aufweist und der vorgegebene Volumenfluss erzielt wird. Bevorzugt ist das Steuergerät 4 im Inneren des Beatmungsgeräts 1 angeordnet.

Im Ausführungsbeispiel sind

- am Inspirations-Anschlussstück 50 für die Inspirationsleitung 30 ein Inspirations-Anzeigeelement 40,
- am Exspirations-Anschlussstück 51 für die Exspirationsleitung 31 ein Exspirations-Anzeigeelement 41,
- am Bypass-Anschlussstück 52 für die Bypassleitung 32 ein Bypass-Anzeigeelement 42 und
- an der Zuführ-Fluidführungseinheit 33 ein Anzeigeelement 43

angeordnet. Jedes Anzeigeelement 40, 41, 42, 43 vermag in zumindest einer visuell wahrnehmbaren Weise anzuzeigen, ob ein Fluid mit einem Volumenfluss oberhalb einer vorgegebenen Schranke durch dasjenige Anschlussstück 50, 51, 52 fließt, an welches das Anzeigeelement 40, 41, 42 angebracht oder welchem das Anzeigeelement 40, 41, 42 zugeordnet ist, sowie die Fließrichtung dieses Fluids, also ob das Fluid aus dem Beatmungsgerät 1 durch das Anschlussstück 50, 51, 52 nach außen oder umgekehrt von außen durch das Anschlussstück 50, 51, 52 in das Beatmungsgerät 1 hineinfließt. Jedes Anzeigeelement 40, 41, 42 weist mindestens einen der beiden möglichen Zustände leuchtend oder nicht leuchtend auf. Das entsprechende gilt für das Anzeigeelement 43.

Optional vermag mindestens ein Anzeigeelement 40, 41, 42, 43 zusätzlich einen Indikator für die Größe (den Betrag) des Volumenflusses durch das zugeordnete Anschlussstück 50, 51, 52, 53 anzuzeigen. Bevorzugt hängt die Helligkeit eines Anzeigeelements 40, 41, 42, 43 vom Volumenfluss ab, und die Helligkeit ist umso größer, je größer der Volumenfluss ist. In einer alternativen Ausgestaltung blinkt oder flackert ein Anzeigeelement 40, 41, 42, 43 mit einer von einem Menschen wahrnehmbaren Frequenz, wobei bevorzugt die Frequenz des Blinkens vom Volumenfluss abhängt und besonders bevorzugt umso größer ist, je größer der Volumenfluss ist. Diese beiden Ausgestaltungen lassen sich miteinander kombinieren.

In einer Realisierungsform wird der ermittelte aktuelle Volumenfluss durch eine Leitung 30, 31, 32, 33 für die nachfolgend beschriebene Anzeige verwendet. In aller Regel variiert der jeweilige Volumenfluss durch eine Leitung 30, 31, 32, 33. Das Steuergerät 4 berechnet in einer alternativen Realisierungsform jeweils einen zeitlich gemittelten Volumenfluss, beispielsweise gemittelt über die Dauer von n Beatmungshüben, wobei n >= 1 eine vorgegebene Anzahl ist, oder als Median über die letzten n Werte. Möglich ist auch, den Volumenfluss über eine vorgegebene Zeitspanne oder auch über die Dauer von n Beatmungshüben numerisch aufzuintegrieren, um die Mittelung zu bilden. Bevorzugt stellt jedes Anzeigeelement 40, 41, 42, 43 jeweils einen Indikator für den gemittelten Volumenfluss dar.

In einer Ausgestaltung steuert das Steuergerät 4 die Anzeigeelemente 40, 41, 42, 43 ausschließlich abhängig von dem ermittelten Maß für den tatsächlichen Volumenfluss durch die jeweilige Fluidführungseinheit an, optional zusätzlich von einem nachfolgenden maximalen Soll-Volumenfluss. In einer anderen Ausgestaltung ermittelt das Steuergerät 4 eine geforderte Fließrichtung durch die jeweilige Fluidführungseinheit hindurch. Beispielsweise fragt das Steuergerät 4 automatisch eine übergeordnete Steuerung oder Regelung des Beatmungssystems ab, wobei diese übergeordnete Steuerung oder Regelung beispielsweise eine Vorgabe eines Benutzers erfasst hat. Dann, wenn die gemessene Fließrichtung von der geforderten Fließrichtung abweicht, insbesondere dann, wenn überhaupt kein Fluid oberhalb einer vorgegebenen Mindest-Volumenfluss-Schranke fließt, wird das jeweilige Anzeigeelement 40, 41, 42, 43 hervorgehoben gekennzeichnet.

Das Steuergerät 4 steuert die Anzeigeelemente 40, 41, 42, 43 an, und zwar abhängig von Signalen der Volumenfluss-Sensoren 10 und 11 und von weiteren Signalen, was nachfolgend beschrieben wird. Das Steuergerät 4 vermag den Zustand jedes Anzeigeelements 40, 41, 42, 43 unabhängig vom jeweiligen Zustand jedes anderen Anzeigeelements einzustellen und zu verändern.

In einer Ausgestaltung umfasst jedes Anzeigeelement 40, 41, 42, 43 jeweils mindestens eine LED oder ein sonstiges geeignetes Leuchtmittel. An jedem Anzeigeelement 40, 41, 42, 43 liegt eine gepulste elektrische Spannung an. Das Steuergerät 4 verändert bevorzugt die jeweilige Helligkeit jedes Anzeigeelements 40, 41, 42, 43 durch eine Pulsweitenmodulation (PWM). Hierbei wird die Länge der elektrischen Pulse und / oder die Pause zwischen zwei Pulsen eingestellt und bei Bedarf verändert. Selbstverständlich kann das Steuergerät 4 ein Anzeigeelement 40, 41, 42, 43 auch ausschalten.

In einer Ausgestaltung realisiert das Beatmungssystem einen Beatmungskreislauf, vgl. Figur 2. Erfindungsgemäß ermittelt das Steuergerät 4 einen Netto-Inspirations-Volumenfluss $Vol'_{insp}(50)$, also den Volumenfluss, den die Antriebseinheit 7 mit den Beatmungshüben erzielt. Der gesamte Volumenfluss $Vol'(30)$ durch die Inspirationsleitung 30 umfasst eine Überlagerung dieses Netto-Inspirations-Volumenflusses $Vol'_{insp}(50)$ und desjenigen Volumenflusses, der dadurch entsteht, dass das vom Patienten Pt ausgeatmete Exspirations-Gasgemisch wieder zur Inspirationsleitung 30 geleitet wird, nachdem Kohlendioxid herausgefiltert ist. Beispielsweise verwendet das Steuergerät 4 Signale von den beiden Volumenfluss-Sensoren 10 und 11, vom Antriebseinheits-Sensor 8 sowie vom optionalen $CO_2$-Sensor 15 oder von einem nicht gezeigten Sensor, der ein Maß für die Menge des herausgefilterten Kohlendioxid misst, um den Netto-Inspirations-Volumenfluss $Vol'_{insp}(50)$ zu ermitteln. Die nachfolgende beispielhafte Beschreibung bezieht sich auf eine Anwendung, bei der ein Beatmungskreislauf hergestellt ist.

In einer anderen Ausgestaltung fließt Atemluft aus dem Gasmischer 6 durch die Bypassleitung 32 direkt zur patientenseitigen Koppeleinheit 9. Bevorzugt wird bei dieser Ausgestaltung die Luft, die der Patient Pt ausatmet, in die Umgebung abgegeben, vgl. Figur 1.

In einer Ausgestaltung fließt durch mindestens eine Leitung 30, 31, 32, 33 zusätzlich ein Gas, welches nicht zum oder vom Patienten Pt fließt, sondern beispielsweise für eine Reinigung der Leitung 30, 31, 32, 33 oder für die Messung eines Gas-Bestandteils in einem Gasgemisch verwendet wird. Das Steuergerät 4 kompensiert rechnerisch den Einfluss dieses Volumenflusses auf den Inspirations-Volumenfluss oder den Exspirations-Volumenfluss.

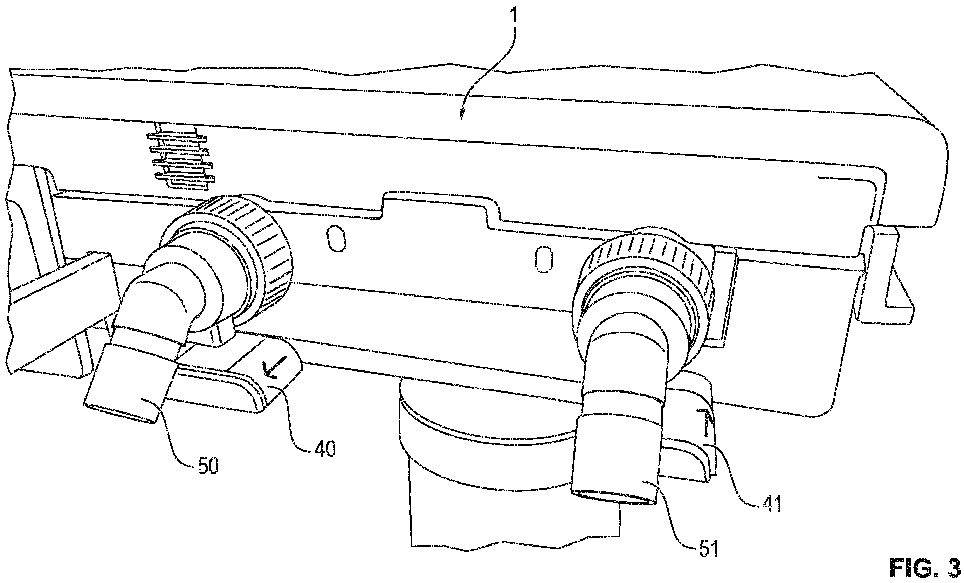

Figur 3 zeigt beispielhaft das Anzeigeelement 40 am Inspirations-Anschlussstück 50 und das Anzeigeelement 41 am Exspirations-Anschlussstück 51. Auf diese Anschlussstücke 50, 51 lässt sich jeweils ein Schlauch aufstecken und wieder abziehen. Jedes Anzeigeelement 40, 41 ist abhängig von der Ansteuerung durch das Steuergerät 4 leuchtend oder nicht leuchtend. Ein Pfeil auf dem Anzeigeelement 40, 41 zeigt die aktuelle Fließrichtung von Fluid durch das jeweilige Anschlussstück 50, 51 an. Optional ist ein Anzeigeelement 40, 41 umso heller, je größer der Volumenfluss durch das Anschlussstück 50, 51 ist.

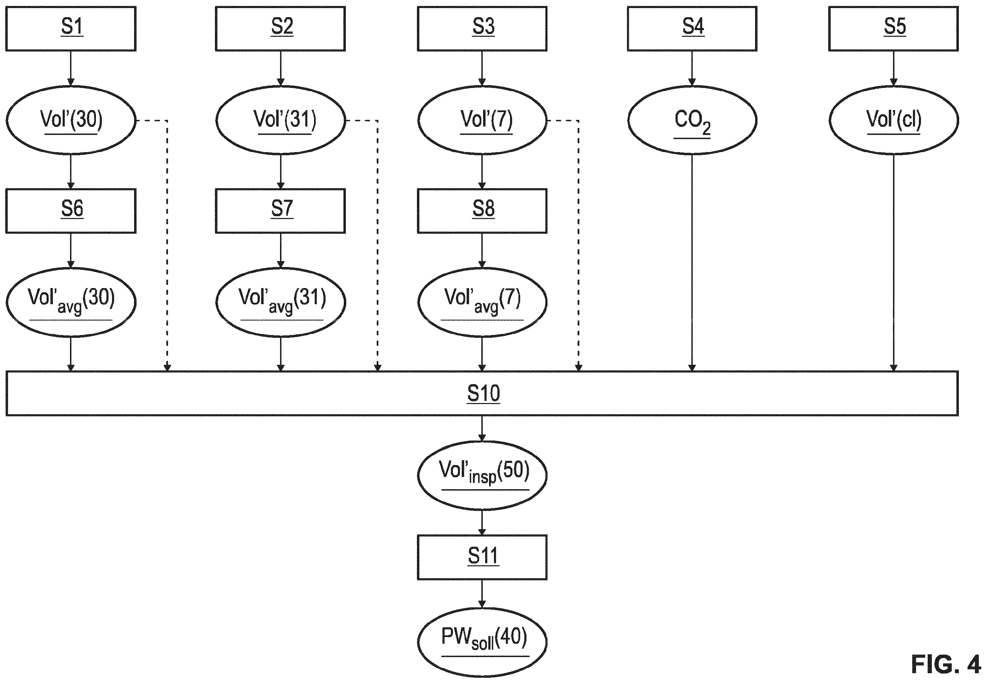

Figur 4 veranschaulicht mit Hilfe eines Flussdiagramms beispielhaft, wie automatisch eine Vorgabe für die Helligkeit hergeleitet wird, wobei das Anzeigeelement 40 am Inspirations-Anschlussstück 50 mithilfe der hergeleiteten Helligkeit denjenigen Anteil des Volumenfluss durch das Inspirations-Anschlussstück 50 visualisiert, der von der Antriebseinheit 7 erzeugt wird, also den Netto-Inspirations-Volumenfluss $Vol'_{insp}(50)$. Hierbei bedeuten:

| | | |
|---|---|---|
| S1 | Schritt: Der Volumenfluss-Sensor 10 misst den Volumenfluss $Vol'(30)$ durch die Inspirationsleitung 30. | |
| S2 | Schritt: Der Volumenfluss-Sensor 11 misst den Volumenfluss $Vol'(31)$ durch die Exspirationsleitung 31. | |
| S3 | Schritt: Der Antriebseinheits-Sensor 8 misst ein Maß für den Volumenfluss $Vol'(7)$, den die Antriebseinheit 7 erzeugt. | |
| S4 | Schritt: Der $CO_2$-Sensor 15 misst ein Maß für die Konzentration $CO_2$ von Kohlendioxid im Exspirations-Gasgemisch, das durch die Exspirationsleitung 31 fließt. | |
| S5 | Schritt: Ein Maß für den gemittelten Spül- und / oder Mess-Volumenfluss $Vol'(cl)$ wird ermittelt. | |
| S6 | Schritt: Das Steuergerät 4 berechnet einen zeitlich gemittelten Volumenfluss $Vol'_{avg}(30)$ durch die Inspirationsleitung 30. | |
| S7 | Schritt: Das Steuergerät 4 berechnet einen zeitlich gemittelten Volumenfluss $Vol'_{avg}(31)$ durch die Exspirationsleitung 31. | |
| S8 | Schritt: Das Steuergerät 4 berechnet einen zeitlich gemittelten Volumenfluss $Vol'_{avg}(7)$, den die Antriebseinheit 7 erzeugt. | |

(fortgesetzt)

| | | |
|---|---|---|
| S10 | Schritt: Das Steuergerät 4 berechnet den gemittelten Netto-Inspirations-Volumenfluss $Vol'_{insp}(50)$ durch das Inspirations-Anschlussstück 50, den die Antriebeinheit 7 erzeugt und der vom Anzeigeelement 40 visualisiert wird. | |
| S11 | Schritt: Das Steuergerät 4 berechnet eine Soll-Pulsweite $PW_{soll}(40)$ für das Anzeigeelement 40. | |

Anmerkung: Falls kein Fehler auftritt, ist der gemittelte Netto-Inspirations-Volumenfluss $Vol'_{insp}(50)$ idealerweise gleich dem gemittelten Volumenfluss $Vol'_{avg}(7)$.

Die Schritte S6, S7 und S8 sind optionale Schritte. In Figur 4 ist durch gestrichelte Pfeile angedeutet, wie der Ablauf ist, wenn diese Schritte fortgelassen werden.

Bevorzugt ermittelt das Steuergerät 4 im Schritt S10 den Netto-Inspirations-Volumenfluss $Vol'_{insp}(50)$ aufgrund der Volumenflüsse $Vol'(30)$, $Vol'(31)$ und $Vol'(cl)$. Bevorzugt wird vorausgesetzt, dass alles Kohlendioxid aus dem Exspirations-Gasgemisch herausgefiltert wird und das Exspirations-Gasgemisch ohne Kohlendioxid vollständig wieder der Inspirationsleitung 30 zugeführt wird. Dann gilt:

$$Vol'(30) = Vol'_{insp}(50) + (1-CO_2)*Vol'(31) + Vol'(cl).$$

Hieraus resultiert die Rechenvorschrift

$$Vol'_{insp}(50) = Vol'(30) - (1-CO_2)*Vol'(31) - Vol'(cl).$$

Der Volumenfluss $Vol'_{avg}(7)$ wird für eine Plausibilitätsprüfung verwendet, die das Steuergerät 4 automatisch durchführt. Idealerweise gilt

$$Vol'_{avg}(7) = Vol'(30) - (1-CO_2)*Vol'(31) - Vol'(cl).$$

Bei einer erheblichen Abweichung zwischen der linken und der rechten Seite dieser Gleichung liegt ein Fehler vor, beispielsweise ein Messfehler oder ein Ausfall eines Sensors.

In einer Ausgestaltung wird die Tatsache berücksichtigt, dass der maximale Soll-Volumenfluss, den die Antriebeinheit 7 mit den Beatmungshüben erzielen soll, von Patient zu Patient unterschiedlich sein kann. Insbesondere ist der maximale Soll-Volumenfluss bei einem Kind deutlich geringer als bei einem Erwachsenen. Bevorzugt wird dieser Soll-Volumenfluss berücksichtigt, um folgenden unerwünschten Effekt zu verhindern: Ohne Berücksichtigung des Soll-Volumenflusses würde bei einem relativ geringen maximalen Soll-Volumenfluss ein Anzeigeelement 40, 41, 42, 43 nur schwach und / oder mit einer geringen Frequenz leuchten, selbst wenn der zeitlich gemittelte tatsächliche Netto-Inspirations-Volumenfluss $Vol'_{insp}(50)$ den maximale Soll-Volumenfluss erreicht. Insbesondere bei einer erheblichen Umgebungsbeleuchtung wäre der Zustand des Anzeigeelements 40, 41, 42, 43 dann nur relativ schwer zu erfassen. Man könnte fälschlicherweise zum Ergebnis kommen, dass überhaupt kein oder zu wenig Fluid fließt.

Bevorzugt erfasst das Steuergerät 4 ein Maß für den maximalen Soll-Volumenfluss, beispielsweise von einer übergeordneten Steuerung oder Regelung, die einen zeitlichen Verlauf des Soll-Volumenflusses durch die Inspirationsleitung 30 vorgibt, oder aufgrund einer Vorgabe eines Benutzers. In einer einfachen Ausgestaltung erfasst und / oder ermittelt das Steuergerät 4 das Gewicht und / oder das Lebensalter und / oder Geburtsdatum des Patienten Pt, beispielsweise aus Daten über den Patienten Pt, die ein Benutzer eingegeben hat, oder aus einer Datenbank mit Patientendaten. Zu diesen Daten gehören insbesondere das Alter, das Gewicht und die Größe des Patienten Pt. Das Steuergerät 4 leitet aus erfassten Daten über den Patienten Pt unter Verwendung einer vorgegebenen Tabelle einen groben Schätzwert für den maximalen Soll-Volumenfluss her. Die Helligkeit und / oder die Frequenz, mit der die beiden Anzeigeelemente 40 und 41 leuchten und / oder flackern, hängt vom Quotienten aus dem ermittelten tatsächlichen Netto-Inspirations-Volumenfluss $Vol'_{insp}(50)$ und dem erfassten maximalen Soll-Volumenfluss bei der Beatmung eines bestimmten Patienten Pt ab. Die Helligkeit und / oder Frequenz des Anzeigeelements 41 am Exspirations-Anschlussstück 51 hängt in einer Ausgestaltung vom Quotienten zwischen dem zeitlich gemittelten Volumenfluss $Vol'_{avg}(31)$ durch die Exspirationsleitung 31 und dem maximalen Soll-Volumenfluss ab.

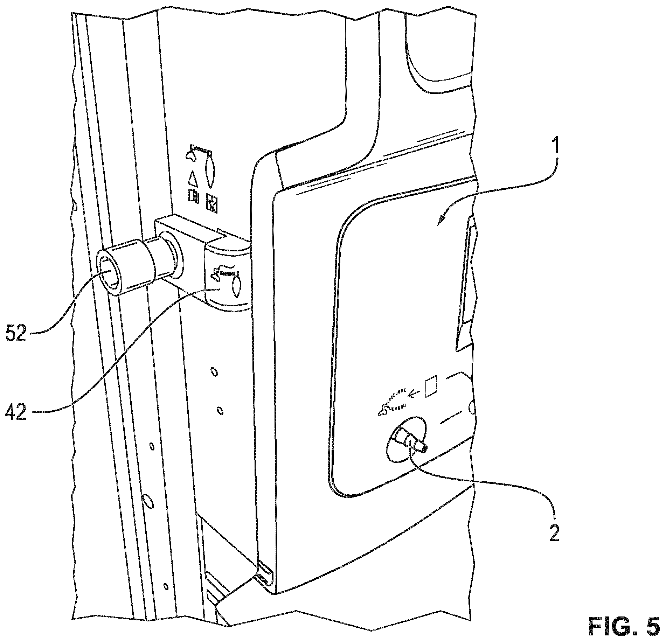

Figur 5 zeigt das Bypass-Anzeigeelement 42 am Bypass-Anschlussstück 52. In einer Ausgestaltung misst ein weiterer Volumenfluss-Sensor (nicht gezeigt) den Volumenfluss in oder durch die Bypassleitung 32, und die Helligkeit des Bypass-Anzeigeelements 42 hängt vom gemessenen Volumenfluss ab. In einer anderen Ausgestaltung hängt die Helligkeit des Bypass-Anzeigeelements 42 vom gewünschten Volumenfluss ab, der mit Hilfe der Eingabeeinheit 12 vorgegeben wird. Möglich ist auch, dass das Bypass-Anzeigeelement 42 nur zwei mögliche Zustände aufweist, nämlich leuchtend und nicht leuchtend. In einer Ausgestaltung leuchtet das Anzeigeelement 42 nur dann, wenn der Schalter 2 so eingeschaltet ist, dass er ein Gas aus dem Gasmischer 6 in die Bypassleitung 32 leitet. In einer anderen Ausgestaltung hängt der aktuelle Zustand des Bypass-Anzeigeelements 42 nur vom gemessenen oder vorgegebenen Volumenfluss ab, aber nicht von der Stellung des Schalters 2.

Bezugszeichenliste

| | |
|---|---|
| 1 | Beatmungsgerät, umfasst die Antriebseinheit 7 mit der Pumpe 5, die Eingabeeinheit 12, den Schalter 2, die Volumenfluss-Sensoren 10 und 11 und die Anzeigeelemente 40, 41 und 42 |
| 2 | pneumatischer Schalter, um das Gasgemisch wahlweise zum Beatmungsgerät 1 oder direkt durch die Bypassleitung 32 hindurch zur patientenseitigen Koppeleinheit 9 zu leiten |
| 3 | optionaler Handbeatmungsbeutel, pneumatisch mit der Bypassleitung 32 verbunden |
| 4 | signalverarbeitendes Steuergerät, empfängt Signale von den Volumenfluss-Sensoren 10 und 11, den Sensoren 8 und 15, den Eingabeeinheiten 12 und 13 und dem Schalter 2, steuert die Anzeigeelemente 40, 41 und 42 sowie den Gasmischer 6 an |
| 5 | Fluidfördereinheit in Form einer Pumpe, gehört zur Antriebseinheit 7 |
| 6 | Gasmischer, erzeugt ein Gasgemisch aus reinem Sauerstoff, Atemluft und Lachgas (N2O), ist mit den Versorgungsanschlüssen 20, 21, 22 und mit dem Schalter 2 verbunden |
| 7 | Antriebseinheit des Beatmungsgeräts 1, umfasst die Pumpe 5 |
| 8 | Antriebseinheits-Sensor, der ein Maß für den Volumenfluss misst, den die Antriebseinheit 7 erzeugt |
| 9 | patientenseitige Koppeleinheit in Form einer Atemmaske, auf dem Gesicht des Patienten Pt angeordnet |
| 10 | Inspirations-Volumenfluss-Sensor, misst einen Druckunterschied am pneumatischen Widerstand R.2 als Maß für den Volumenfluss durch die Inspirationsleitung 30 |
| 11 | Exspirations-Volumenfluss-Sensor, misst einen Druckunterschied am pneumatischen Widerstand R.1 als Maß für den Volumenfluss durch die Exspirationsleitung 31 |
| 12 | Eingabeeinheit am Beatmungsgerät 1, mit dem ein Benutzer einen gewünschten Volumenfluss des Gasgemischs, welches der Gasmischer 6 bereitstellen soll, vorgibt |
| 13 | Eingabeeinheit am Gasmischer 6, mit dem ein Benutzer ein gewünschtes Mischungsverhältnis im Gasgemisch, welches der Gasmischer 6 erzeugen soll, vorgibt |
| 15 | Kohlendioxid-Sensor, misst ein Maß für die Konzentration (den Anteil) von Kohlendioxid im Exspirations-Gasgemisch, das durch die Exspirationsleitung 31 fließt |
| 20 | Versorgungsanschluss für reinen Sauerstoff in der Wand W |
| 21 | Versorgungsanschluss für Atemluft in der Wand W |
| 22 | Versorgungsanschluss für Lachgas (N2O) in der Wand W |
| 30 | Inspirationsleitung, leitet ein Gasgemisch vom Beatmungsgerät 1 zur patientenseitigen Koppeleinheit 9 |
| 31 | Exspirationsleitung, leitet ausgeatmete Luft von der patientenseitigen Koppeleinheit 9 zum Beatmungsgerät 1 |
| 32 | Bypassleitung, leitet ein Gasgemisch vom Schalter 2 in die Inspirationsleitung 30 oder zur patientenseitigen Koppeleinheit 9 |
| 33 | Zuführ-Fluidführungseinheit, leitet ein Gasgemisch vom Gasmischer 6 zum Schalter 2 |
| 40 | Anzeigeelement am Inspirations-Anschlussstück 50 für die Inspirationsleitung 30, wird mit der Soll-Pulsweite $PW_{soll}(40)$ betrieben |
| 41 | Anzeigeelement am Exspirations-Anschlussstück 51 für die Exspirationsleitung 31 |
| 42 | Anzeigeelement am Bypass-Anschlussstück 52 für die Bypassleitung 32 |

| 43 | Anzeigeelement an der Zuführ-Fluidführungseinheit 33 |
|---|---|
| 50 | Inspirations-Anschlussstück, an das sich die Inspirationsleitung 30 anschließen lässt, mit dem Anzeigeelement 40 verbunden |
| 51 | Exspirations-Anschlussstück, an das sich die Exspirationsleitung 31 anschließen lässt, mit dem Anzeigeelement 41 verbunden |
| 52 | Bypass-Anschlussstück, an das sich die Bypassleitung 32 anschließen lässt, mit dem Anzeigeelement 42 verbunden |
| $CO_2$ | Anteil an Kohlendioxid im Exspirations-Gasgemisch, das durch die Exspirationsleitung 31 fließt, vom Sensor 15 gemessen |
| $\Delta P.1$ | Druckdifferenz am pneumatischen Widerstand R.1, vom Sensor 11 gemessen |
| $\Delta P.2$ | Druckdifferenz am pneumatischen Widerstand R.2, vom Sensor 10 gemessen |
| Pt | Patient, der künstlich beatmet und in einer Ausgestaltung narkotisiert wird, trägt auf seinem Gesicht die patientenseitige Koppeleinheit 9 |
| $PW_{soll}(40)$ | vom Steuergerät 4 berechnete Soll-Pulsweite, legt die Helligkeit des Anzeigeelements 40 fest |
| R.1 | pneumatischer Widerstand in der Exspirationsleitung 31 |
| R.2 | pneumatischer Widerstand in der Inspirationsleitung 30 |
| S1 | Schritt: Der Volumenfluss-Sensor 10 misst den Volumenfluss Vol'(30) durch die Inspirationsleitung 30 |
| S2 | Schritt: Der Volumenfluss-Sensor 11 misst den Volumenfluss Vol'(31) durch die Exspirationsleitung 31 |
| S3 | Schritt: Der Antriebseinheits-Sensor 8 misst ein Maß für den Volumenfluss Vol'(7), den die Antriebseinheit 7 erzeugt |
| S4 | Schritt: Der $CO_2$-Sensor 15 misst ein Maß für die Konzentration von $CO_2$ von Kohlendioxid im Exspirations-Gasgemisch, das durch die Exspirationsleitung 31 fließt |
| S5 | Schritt: Ein Maß für den gemittelten Spül- und/oder Mess-Volumenfluss Vol'(cl) wird ermittelt |
| S6 | optionaler Schritt: Das Steuergerät 4 berechnet einen zeitlich gemittelten Volumenfluss Vol'$_{avg}$(30) durch die Inspirationsleitung 30 |
| S7 | optionaler Schritt: Das Steuergerät 4 berechnet einen zeitlich gemittelten Volumenfluss Vol'$_{avg}$(31) durch die Exspirationsleitung 31 |
| S8 | optionaler Schritt: Das Steuergerät 4 berechnet einen zeitlich gemittelten Volumenfluss Vol'$_{avg}$(7), den die Antriebseinheit 7 erzeugt |
| S10 | Schritt: Das Steuergerät 4 berechnet den Netto-Inspirations-Volumenfluss Vol'$_{insp}$(50), optional den gemittelten Volumenfluss, durch das Inspirations-Anschlussstück 50, den die Antriebseinheit 7 erzeugt und vom Anzeigeelement 40 visualisiert wird |
| S11 | Schritt: Das Steuergerät 4 berechnet abhängig vom Netto-Inspirations-Volumenfluss Vol'$_{insp}$(50) eine Soll-Pulsweite $PW_{soll}(40)$ für das Anzeigeelement 50 |
| Vol'(30) | Volumenfluss durch die Inspirationsleitung 30, vom Volumenfluss-Sensor 10 gemessen, umfasst eine Überlagerung des Netto-Inspirations-Volumenflusses Vol'$_{insp}$(50) und des Exspirations-Volumenflusses Vol'(31) sowie optional des Spül- und/oder Mess-Volumenflusses Vol'(cl) |
| Vol'(31) | Volumenfluss durch die Exspirationsleitung 31, vom Volumenfluss-Sensor 11 gemessen |
| Vol'(7) | Volumenfluss, den die Antriebseinheit 7 erzeugt, vom Antriebseinheits-Sensor 8 gemessen |
| Vol'(cl) | Volumenfluss, der beim Spülen der Inspirationsleitung 30 und optional beim Abzweigen einer Gasprobe aus der Inspirationsleitung 30 entsteht |
| Vol'$_{avg}$(30) | zeitlich gemittelter Volumenfluss durch die Inspirationsleitung 30, ist eine Überlagerung des gemittelten Netto-Inspirations-Volumenflusses Vol'$_{insp}$(50) und des gemittelten Exspirations-Volumenflusses Vol'$_{avg}$(31) |

(fortgesetzt)

| Vol'$_{avg}$(31) | zeitlich gemittelter Exspirations-Volumenfluss durch die Exspirationsleitung 31 |
|---|---|
| Vol'$_{avg}$(7) | zeitlich gemittelter Volumenfluss, den die Antriebseinheit 7 erzeugt |
| Vol'$_{insp}$(50) | zeitlich gemittelter Netto-Inspirations-Volumenfluss durch das Inspirations-Anschlussstück 50, vom Steuergerät 4 ermittelt |
| W | Wand, in der die Versorgungsanschlüsse 20, 21, 22 eingelassen sind |

**Patentansprüche**

1. Beatmungssystem für die künstliche Beatmung eines Patienten (Pt), wobei das Beatmungssystem

- ein Beatmungsgerät (1),
- eine Inspirations-Fluidführungseinheit (30),
- eine patientenseitige Koppeleinheit (9),
- ein signalverarbeitendes Steuergerät (4) und
- ein Inspirations-Anzeigeelement (40),

umfasst,
wobei die patientenseitige Koppeleinheit (9) wenigstens zeitweise mit dem Patienten (Pt) verbunden oder verbindbar ist,
wobei das Beatmungsgerät (1) dazu ausgestaltet ist, ein Gasgemisch umfassend Sauerstoff auszustoßen,
wobei die Inspirations-Fluidführungseinheit (30) dazu ausgestaltet ist, ein vom Beatmungsgerät (1) ausgestoßenes Gasgemisch zur patientenseitigen Koppeleinheit (9) zu leiten,
wobei das Inspirations-Anzeigeelement (40)

- an der Inspirations-Fluidführungseinheit (30) oder
- an einer anderen Fluidführungseinheit (50), die wenigstens zeitweise in einer Fluidverbindung mit dem Beatmungsgerät (1) und mit der Inspirations-Fluidführungseinheit (30) steht,

befestigt ist,
wobei das Steuergerät (4) dazu ausgestaltet ist,

- ein Maß für den Netto-Inspirations-Volumenfluss [Vol'$_{insp}$(50)] zu ermitteln,

wobei der Netto-Inspirations-Volumenfluss [Vol'$_{insp}$(50)]
der Volumenfluss durch die Inspirations-Fluidführungseinheit (30) hindurch ist, welchen das Beatmungsgerät (1) durch das Ausstoßen des Gasgemischs erzeugt, und
ein Teil des gesamten Volumenflusses durch die Inspirations-Fluidführungseinheit (30) hindurch ist, und

- das Inspirations-Anzeigeelement (40) abhängig vom ermittelten Netto-Inspirations-Volumenfluss [Vol'$_{insp}$(50)] anzusteuern, und
wobei das Inspirations-Anzeigeelement (40) dazu ausgestaltet ist, abhängig von der Ansteuerung in mindestens einer visuell wahrnehmbaren Weise
- einen Indikator für die Größe des ermittelten Netto-Inspirations-Volumenflusses [Vol'$_{insp}$(50)] und
- einen Indikator für die Fließrichtung des Gasgemischs durch die Inspirations-Fluidführungseinheit (30) hindurch

anzuzeigen.

2. Beatmungssystem nach Anspruch 1,
**dadurch gekennzeichnet, dass**

das Beatmungssystem einen Inspirations-Volumenfluss-Sensor (10) umfasst,
wobei der Inspirations-Volumenfluss-Sensor (10) dazu ausgestaltet ist, ein Maß für den Volumenfluss [Vol'(30)] durch die Inspirations-Fluidführungseinheit (30) hindurch zu messen, und
wobei das Steuergerät (4) dazu ausgestaltet ist, das Maß für den Netto-Inspirations-Volumenfluss [Vol'$_{insp}$(50)]

unter Verwendung eines Signals des Inspirations-Volumenfluss-Sensors (10) zu ermitteln.

3. Beatmungssystem nach Anspruch 2,
   **dadurch gekennzeichnet, dass**
   das Steuergerät (4) dazu ausgestaltet ist,

   - im Signal des Inspirations-Volumenfluss-Sensors (10) einen oszillierenden Anteil zu ermitteln und
   - die Frequenz und / oder die Amplitude des oszillierenden Anteils zu ermitteln und
   - das Maß für den Netto-Inspirations-Volumenfluss [Vol'$_{insp}$(50)] unter Verwendung der ermittelten Frequenz und / oder Amplitude zu ermitteln.

4. Beatmungssystem nach Anspruch 1,
   **dadurch gekennzeichnet, dass**

   das Beatmungsgerät (1) weiterhin ein Inspirations-Anschlussstück (50) umfasst,
   wobei die Inspirations-Fluidführungseinheit (30) dazu ausgestaltet ist, lösbar und fluiddicht mit dem Inspirations-Anschlussstück (50) verbunden zu werden,
   wobei nach dem Verbunden-Werden das Inspirations-Anschlussstück (50) eine Fluidverbindung zwischen der Inspirations-Fluidführungseinheit (30) und dem Beatmungsgerät (1) herstellt und
   wobei das Inspirations-Anzeigeelement (40) am Inspirations-Anschlussstück (50) befestigt ist.

5. Beatmungssystem nach einem der vorhergehenden Ansprüche,
   **dadurch gekennzeichnet, dass**

   das Beatmungssystem weiterhin

   - eine Exspirations-Fluidführungseinheit (31) und
   - ein Exspirations-Anzeigeelement (41), das vom Inspirations-Anzeigeelement (40) räumlich beabstandet ist,

   umfasst und
   wobei die Exspirations-Fluidführungseinheit (31) dazu ausgestaltet ist, ein aus der patientenseitigen Koppeleinheit (9) austretendes Gasgemisch zum Beatmungsgerät (1) zu leiten,
   wobei das Exspirations-Anzeigeelement (41)

   - an der Exspirations-Fluidführungseinheit (31) oder
   - an einer anderen Fluidführungseinheit (51), die wenigstens zeitweise in einer Fluidverbindung mit dem Beatmungsgerät (1) und mit der Exspirations-Fluidführungseinheit (31) steht,

   befestigt ist,
   wobei das Steuergerät (4) dazu ausgestaltet ist,

   - ein Maß für den Exspirations-Volumenfluss [Vol'$_{avg}$(31)] zu ermitteln, wobei der Exspirations-Volumenfluss [Vol'$_{avg}$(31)] der Volumenfluss ist, welcher durch die Exspirations-Fluidführungseinheit (31) hindurch zum Beatmungsgerät (1) fließt, und
   - das Exspirations-Anzeigeelement (41) abhängig vom ermittelten Exspirations-Volumenfluss [Vol'$_{avg}$(31)] anzusteuern, und

   wobei das Exspirations-Anzeigeelement (41) dazu ausgestaltet ist, abhängig von der Ansteuerung in mindestens einer visuell wahrnehmbaren Weise

   - einen Indikator für die Größe des ermittelten Exspirations-Volumenflusses [Vol'$_{avg}$(31)] und
   - einen Indikator für die Fließrichtung des Gasgemischs, welches durch die Exspirations-Fluidführungseinheit (31) hindurch fließt,

   anzuzeigen.

6. Beatmungssystem nach Anspruch 4 und nach Anspruch 5,

**EP 4 414 013 B1**

**dadurch gekennzeichnet, dass**

das Beatmungsgerät (1) weiterhin ein Exspirations-Anschlussstück (51) umfasst, das vom Inspirations-Anschlussstück (50) räumlich beabstandet ist,
wobei die Exspirations-Fluidführungseinheit (31) dazu ausgestaltet ist, lösbar und fluiddicht mit dem Exspirations-Anschlussstück (51) verbunden zu werden,
wobei nach dem Verbunden-Werden das Exspirations-Anschlussstück (51) eine Fluidverbindung zwischen der Exspirations-Fluidführungseinheit (31) und dem Beatmungsgerät (1) herstellt und
wobei das Exspirations-Anzeigeelement (41) am Exspirations-Anschlussstück (51) befestigt ist.

7. Beatmungssystem nach Anspruch 5 oder Anspruch 6,
**dadurch gekennzeichnet, dass**

das Beatmungssystem

- einen Inspirations-Volumenfluss-Sensor (10) und
- einen Exspirations-Volumenfluss-Sensor (11)

umfasst,
wobei der Inspirations-Volumenfluss-Sensor (10) dazu ausgestaltet ist, ein Maß für den Volumenfluss [Vol'(30)] durch die Inspirations-Fluidführungseinheit (30) hindurch zu messen,
wobei der Exspirations-Volumenfluss-Sensor (11) dazu ausgestaltet ist, ein Maß für den Volumenfluss [Vol'(31)] durch die Exspirations-Fluidführungseinheit (31) hindurch zu messen,
wobei das Beatmungssystem dazu ausgestaltet ist, wenigstens zeitweise einen Beatmungskreislauf zwischen der patientenseitigen Koppeleinheit (9) und dem Beatmungsgerät (1) zu erzeugen,
wobei der Beatmungskreislauf durch die Inspirations-Fluidführungseinheit (30) und durch die Exspirations-Fluidführungseinheit (31) hindurchführt und
wobei das Steuergerät (4) dazu ausgestaltet ist, das Maß für den Netto-Inspirations-Volumenfluss [Vol'$_{insp}$(50)]

- unter Verwendung des gemessenen Volumenflusses [Vol'(30)] durch die Inspirations-Fluidführungseinheit (30) und
- unter Verwendung des gemessenen Volumenflusses [Vol'(31)] durch die Exspirations-Fluidführungseinheit (31)

zu ermitteln.

8. Beatmungssystem nach einem der vorhergehenden Ansprüche,
**dadurch gekennzeichnet, dass**
das Steuergerät (4) dazu ausgestaltet ist,

- ein Maß für einen vorgegebenen maximalen Soll-Volumenfluss, welchen das Beatmungsgerät (1) durch das Ausstoßen des Gasgemischs erzeugen soll, zu erfassen und
- das Inspirations-Anzeigeelement (40) abhängig vom Quotienten zwischen dem ermittelten Netto-Inspirations-Volumenfluss [Vol'$_{insp}$(50)] und dem erfassten maximalen Soll-Volumenfluss anzusteuern.

9. Beatmungssystem nach einem der vorhergehenden Ansprüche,
**dadurch gekennzeichnet, dass**

der angezeigte Indikator für den ermittelten Netto-Inspirations-Volumenfluss [Vol'$_{insp}$(50)] eine Helligkeit und / oder eine Frequenz eines Blinkens oder Flackerns des Inspirations-Anzeigeelements (40) umfasst,
wobei das Beatmungssystem so ausgestaltet ist, dass die Helligkeit und / oder die Frequenz vom ermittelten Netto-Inspirations-Volumenfluss [Vol'$_{insp}$(50)] abhängt.

10. Beatmungssystem nach einem der vorhergehenden Ansprüche,
**dadurch gekennzeichnet, dass**

das Beatmungssystem

- eine Bypass-Fluidführungseinheit (32),
- ein Bypass-Anzeigeelement (42) und
- einen pneumatischen Schalter (2)

umfasst,
wobei die Bypass-Fluidführungseinheit (32) einen Ausgang des Schalters (2) mit der patientenseitigen Koppeleinheit (9) verbindet,
wobei der Schalter (2) dazu ausgestaltet ist, ein Gemisch wahlweise in das Beatmungsgerät (1) oder in die Bypass-Fluidführungseinheit (32) zu leiten,
wobei das Bypass-Anzeigeelement (42)

- an der Bypass-Fluidführungseinheit (32) oder
- an einer anderen Fluidführungseinheit (52), die wenigstens zeitweise in einer Fluidverbindung mit dem Beatmungsgerät (1) und mit der Bypass-Fluidführungseinheit (32) steht,

befestigt ist,
wobei das Steuergerät (4) dazu ausgestaltet ist,

- ein Maß für den Bypass-Volumenfluss, das ist der Volumenfluss durch die Bypass-Fluidführungseinheit (32) hindurch, zu ermitteln und
- das Bypass-Anzeigeelement (42) abhängig vom ermittelten Bypass-Volumenfluss anzusteuern, und

wobei das Bypass-Anzeigeelement (42) dazu ausgestaltet ist, abhängig von der Ansteuerung in mindestens einer visuell wahrnehmbaren Weise

- einen Indikator für die Größe des ermittelten Bypass-Volumenflusses und
- optional einen Indikator für die Fließrichtung des Gasgemischs durch die Bypass-Fluidführungseinheit (32) hindurch

anzuzeigen.

11. Beatmungssystem nach einem der vorhergehenden Ansprüche,
**dadurch gekennzeichnet, dass**

das Steuergerät (4) dazu ausgestaltet ist,

- ein Maß für einen geforderten Netto-Inspirations-Volumenfluss durch die Inspirations-Fluidführungseinheit (30) hindurch zu erfassen,
insbesondere durch eine Anfrage an eine Steuerung oder Regelung des Beatmungsgeräts (1), und
- das Inspirations-Anzeigeelement (40) zusätzlich abhängig vom erfassten geforderten Netto-Inspirations-Volumenfluss anzusteuern, und

das Inspirations-Anzeigeelement (40) dazu ausgestaltet ist, mindestens einen der beiden Indikatoren zusätzlich in Abhängigkeit von dem erfassten geforderten Netto-Inspirations-Volumenfluss anzuzeigen.

**Claims**

1. Ventilation system for artificial ventilation of a patient (Pt),

wherein the ventilation system comprises

- a ventilation device (1),
- an inspiratory fluid guide unit (30),
- a patient-side coupling unit (9),
- a signal-processing control device (4) and
- an inspiratory display element (40),

wherein the patient-side coupling unit (9) is at least temporarily connected or connectable to the patient (Pt),
wherein the ventilation device (1) is configured to release a gas mixture comprising oxygen,
wherein the inspiratory fluid guide unit (30) is configured to conduct a gas mixture released by the ventilation device (1) to the patient-side coupling unit (9),
wherein the inspiratory display element (40) is attached to

- the inspiratory fluid guide unit (30) or
- another fluid guide unit (50) which is at least temporarily in fluid connection with the ventilation device (1) and with the inspiratory fluid guide unit (30),

wherein the control device (4) is configured to

- determine a measurement for the net inspiratory volume flow [Vol'insp(50)],

wherein the net inspiratory volume flow [$Vol'i_{nsp}(50)$] is
the volume flow through the inspiratory fluid guide unit (30) that the ventilation device (1) generates by releasing the gas mixture and
part of the total volume flow through the inspiratory fluid guide unit (30), and

- control the inspiratory display element (40) on the basis of the determined net inspiratory volume flow [$Vol'_{insp}(50)$], and

wherein the inspiratory display element (40) is configured to display, on the basis of the control, in at least one visually perceptible way,

- an indicator for the variable of the determined net inspiratory volume flow [$Vol'_{insp}(50)$] and
- an indicator for the flow direction of the gas mixture through the inspiratory fluid guide unit (30).

2. Ventilation system according to claim 1,
**characterized in that**

the ventilation system comprises an inspiratory volume flow sensor (10),
the inspiratory volume flow sensor (10) being configured to measure a measurement for the volume flow [Vol'(30)] through the inspiratory fluid guide unit (30), and
the control device (4) being configured to determine the measurement for the net inspiratory volume flow [$Vol'_{insp}$ (50)] using a signal of the inspiratory volume flow sensor (10).

3. Ventilation system according to claim 2,
**characterized in that**
the control device (4) is configured to

- determine an oscillating component in the signal of the inspiratory volume flow sensor (10) and
- determine the frequency and/or the amplitude of the oscillating component and
- determine the measurement for the net inspiratory volume flow [$Vol'_{insp}(50)$] using the determined frequency and/or amplitude

4. Ventilation system according to claim 1,
**characterized in that**

the ventilation device (1) further comprises an inspiratory connecting piece (50),
the inspiratory fluid guide unit (30) being configured to be detachably and fluid-tightly connected to the inspiratory connecting piece (50),
the inspiratory connecting piece (50) establishing a fluid connection between the inspiratory fluid guide unit (30) and the ventilation device (1) after being connected, and
the inspiratory display element (40) being attached to the inspiratory connecting piece (50).

5. Ventilation system according to any of the preceding claims,
**characterized in that**

the ventilation system further comprises

- an expiratory fluid guide unit (31) and
- an expiratory display element (41) which is spatially separated from the inspiratory display element (40),

the expiratory fluid guide unit (31) being configured to conduct a gas mixture exiting the patient-side coupling unit (9) to the ventilation device (1),
the expiratory display element (41) being attached to

- the expiratory fluid guide unit (31) or
- another fluid guide unit (51) which is at least temporarily in fluid connection with the ventilation device (1) and with the expiratory fluid guide unit (31),

the control device (4) being configured to

- determine a measurement for the expiratory volume flow [$Vol'_{avg}(31)$],

the expiratory volume flow [$Vol'_{avg}(31)$] being the volume flow which flows through the expiratory fluid guide unit (31) to the ventilation device (1), and

- control the expiratory display element (41) on the basis of the determined expiratory volume flow [$Vol'_{avg}(31)$],

the expiratory display element (41) being configured to display, on the basis of the control, in at least one visually perceptible way,

- an indicator for the variable of the determined expiratory volume flow [$Vol'_{avg}(31)$] and
- an indicator for the flow direction of the gas mixture which flows through the expiratory fluid guide unit (31).

6. Ventilation system according to claim 4 and claim 5,
**characterized in that**

the ventilation device (1) further comprises an expiratory connecting piece (51) which is spatially separated from the inspiratory connecting piece (50),
the expiratory fluid guide unit (31) being configured to be detachably and fluid-tightly connected to the expiratory connecting piece (51),
the expiratory connecting piece (51) establishing a fluid connection between the expiratory fluid guide unit (31) and the ventilation device (1) after being connected, and
the expiratory display element (41) being attached to the expiratory connecting piece (51).

7. Ventilation system according to claim 5 or claim 6,
**characterized in that**

the ventilation system comprises

- an inspiratory volume flow sensor (10) and
- an expiratory volume flow sensor (11),

the inspiratory volume flow sensor (10) being configured to measure a measurement for the volume flow [$Vol'(30)$] through the inspiratory fluid guide unit (30),
the expiratory volume flow sensor (11) being configured to measure a measurement for the volume flow [$Vol'(31)$] through the expiratory fluid guide unit (31),
the ventilation system being configured to generate, at least temporarily, a ventilation circuit between the patient-side coupling unit (9) and the ventilation device (1),
the ventilation circuit passing through the inspiratory fluid guide unit (30) and through the expiratory fluid guide unit (31) and
the control device (4) being configured to determine the measurement for the net inspiratory volume flow [$Vol'_{insp}(50)$]

- using the measured volume flow [Vol'(30)] through the inspiratory fluid guide unit (30) and
- using the measured volume flow [Vol'(31)] through the expiratory fluid guide unit (31).

**8.** Ventilation system according to any of the preceding claims, **characterized in that**
the control device (4) is configured to

- acquire a measurement for a predetermined maximum target volume flow which the ventilation device (1) is to generate by releasing the gas mixture and
- control the inspiratory display element (40) on the basis of the quotient between the determined net inspiratory volume flow [Vol'$_{insp}$(50)] and the acquired maximum target volume flow.

**9.** Ventilation system according to any of the preceding claims,
**characterized in that**

the displayed indicator for the determined net inspiratory volume flow [Vol'$_{insp}$(50)] comprises a brightness and/or a frequency of a blinking or flickering of the inspiratory display element (40),
the ventilation system being configured such that the brightness and/or the frequency is based on the determined net inspiratory volume flow [Vol'$_{insp}$(50)].

**10.** Ventilation system according to any of the preceding claims,
**characterized in that**

the ventilation system comprises

- a bypass fluid guide unit (32),
- a bypass display element (42) and
- a pneumatic switch (2),

the bypass fluid guide unit (32) connecting an output of the switch (2) to the patient-side coupling unit (9),
the switch (2) being configured to conduct a mixture either into the ventilation device (1) or into the bypass fluid guide unit (32),
the bypass display element (42) being attached to

- the bypass fluid guide unit (32) or
- another fluid guide unit (52) which is at least temporarily in fluid connection with the ventilation device (1) and with the bypass fluid guide unit (32),

the control device (4) being configured to

- determine a measurement indicator for the bypass volume flow, i.e., the volume flow through the bypass fluid guide unit (32), and
- control the bypass display element (42) on the basis of the determined bypass volume flow,

the bypass display element (42) being configured to display, on the basis of the control, in at least one visually perceptible way,

- an indicator for the variable of the determined bypass volume flow and
- optionally, an indicator for the flow direction of the gas mixture through the bypass fluid guide unit (32).

**11.** Ventilation system according to any of the preceding claims,
**characterized in that**

the control device (4) is configured to

- acquire a measurement for a required net inspiratory volume flow through the inspiratory fluid guide unit (30),

in particular by way of a request to an open-loop or closed-loop control system of the ventilation device (1), and

- additionally control the inspiratory display element (40) on the basis of the acquired required net inspiratory volume flow,

the inspiratory display element (40) is configured to display at least one of the two indicators additionally on the basis of the acquired required net inspiratory volume flow.

**Revendications**

1. Système d'assistance respiratoire pour la respiration artificielle d'un patient (Pt),

   dans lequel le système d'assistance respiratoire comprend

   - un appareil d'assistance respiratoire (1),
   - une unité de guidage de fluide d'inspiration (30),
   - une unité de couplage côté patient (9),
   - un appareil de commande (4) traitant les signaux et
   - un élément d'affichage d'inspiration (40),

   dans lequel l'unité de couplage côté patient (9) est reliée ou peut être reliée au moins temporairement au patient (Pt),
   dans lequel l'appareil d'assistance respiratoire (1) est conçu pour expulser un mélange gazeux comprenant de l'oxygène,
   dans lequel l'unité de guidage de fluide d'inspiration (30) est conçue pour conduire un mélange gazeux expulsé par l'appareil d'assistance respiratoire (1) vers l'unité de couplage côté patient (9),
   dans lequel l'élément d'affichage d'inspiration (40) est fixé

   - sur l'unité de guidage de fluide d'inspiration (30) ou
   - sur une autre unité de guidage de fluide (50) qui est au moins temporairement en liaison fluidique avec l'appareil d'assistance respiratoire (1) et avec l'unité de guidage de fluide d'inspiration (30),

   dans lequel l'appareil de commande (4) est configuré pour

   - déterminer une mesure pour le débit volumique d'inspiration net [$Vol'_{insp}$(50)],

   dans lequel le débit volumique d'inspiration net [$Vol'_{insp}$(50)] est
   le débit volumique à travers l'unité de guidage de fluide d'inspiration (30) que l'appareil d'assistance respiratoire (1) produit en expulsant le mélange gazeux, et
   une partie du débit volumique total traverse l'unité de guidage de fluide d'inspiration (30), et

   - commander l'élément d'affichage d'inspiration (40) en fonction du débit volumique d'inspiration net [$Vol'_{insp}$(50)] déterminé, et

   dans lequel l'élément d'affichage d'inspiration (40) est configuré pour afficher, en fonction de la commande, au moins d'une manière perceptible visuellement,

   - un indicateur pour la grandeur du débit volumique d'inspiration net [$Vol'_{insp}$(50)] déterminé et
   - un indicateur pour le sens d'écoulement du mélange gazeux à travers l'unité de guidage de fluide d'inspiration (30).

2. Système d'assistance respiratoire selon la revendication 1,
   **caractérisé en ce que**

   le système d'assistance respiratoire comprend un capteur de débit volumique d'inspiration (10),
   dans lequel le capteur de débit volumique d'inspiration (10) est configuré pour mesurer une mesure pour le débit volumique [$Vol'$(30)] à travers l'unité de guidage de fluide d'inspiration (30), et
   dans lequel l'appareil de commande (4) est configuré pour déterminer la mesure pour le débit volumique d'inspiration net [$Vol'_{insp}$(50)] à l'aide d'un signal du capteur de débit volumique d'inspiration (10).

**3.** Système d'assistance respiratoire selon la revendication 2,
**caractérisé en ce que**
l'appareil de commande (4) est configuré pour

- déterminer une composante oscillante dans le signal du capteur de débit volumique d'inspiration (10) et
- déterminer la fréquence et/ou l'amplitude de la composante oscillante et
- déterminer la mesure pour le débit volumique d'inspiration net [Vol'$_{insp}$(50)] à l'aide de la fréquence et/ou de l'amplitude déterminées.

**4.** Système d'assistance respiratoire selon la revendication 1,
**caractérisé en ce que**

l'appareil d'assistance respiratoire (1) comprend en outre une pièce formant raccord d'inspiration (50),
dans lequel l'unité de guidage de fluide d'inspiration (30) est conçue pour être reliée de manière amovible et étanche aux fluides à la pièce formant raccord d'inspiration (50),
dans lequel, avoir été reliée, la pièce formant raccord d'inspiration (50) établit une liaison fluidique entre l'unité de guidage de fluide d'inspiration (30) et l'appareil d'assistance respiratoire (1) et
dans lequel l'élément d'affichage d'inspiration (40) est fixé sur la pièce formant raccord d'inspiration (50).

**5.** Système d'assistance respiratoire selon l'une des revendications précédentes,
**caractérisé en ce que**

le système d'assistance respiratoire comprend en outre

- une unité de guidage de fluide d'expiration (31) et
- un élément d'affichage d'expiration (41) qui est espacé spatialement de l'élément d'affichage d'inspiration (40),
et

dans lequel l'unité de guidage de fluide d'expiration (31) est conçue pour conduire un mélange gazeux s'échappant de l'unité de couplage côté patient (9) vers l'appareil d'assistance respiratoire (1),
dans lequel l'élément d'affichage d'expiration (41) est fixé

- sur l'unité de guidage de fluide d'expiration (31) ou
- sur une autre unité de guidage de fluide (51) qui est au moins temporairement en liaison fluidique avec l'appareil d'assistance respiratoire (1) et avec l'unité de guidage de fluide d'expiration (31),

dans lequel l'appareil de commande (4) est configuré pour

- déterminer une mesure pour le débit volumique d'expiration [Vol'$_{avg}$(31)],

dans lequel le débit volumique d'expiration [Vol'$_{avg}$(31)] est le débit volumique qui s'écoule à travers l'unité de guidage de fluide d'expiration (31) vers l'appareil d'assistance respiratoire (1), et

- commander l'élément d'affichage d'expiration (41) en fonction du débit volumique d'expiration [Vol'$_{avg}$(31)] déterminé, et

dans lequel l'élément d'affichage d'expiration (41) est configuré pour afficher, en fonction de la commande, au moins d'une manière perceptible visuellement,

- un indicateur pour la grandeur du débit volumique d'expiration [Vol'$_{avg}$(31)] déterminé et
- un indicateur pour le sens d'écoulement du mélange gazeux qui s'écoule à travers l'unité de guidage de fluide d'expiration (31)

**6.** Système d'assistance respiratoire selon la revendication 4 et selon la revendication 5,
**caractérisé en ce que**

l'appareil d'assistance respiratoire (1) comprend en outre une pièce formant raccord d'expiration (51) qui est espacée spatialement de la pièce formant raccord d'inspiration (50),

dans lequel l'unité de guidage de fluide d'expiration (31) est conçue pour être reliée de manière amovible et étanche aux fluides à la pièce formant raccord d'expiration (51),

dans lequel, après avoir été reliée, la pièce formant raccord d'expiration (51) établit une liaison fluidique entre l'unité de guidage de fluide d'expiration (31) et l'appareil d'assistance respiratoire (1) et

dans lequel l'élément d'affichage d'expiration (41) est fixé sur la pièce formant raccord d'expiration (51).

7. Système d'assistance respiratoire selon la revendication 5 ou la revendication 6, **caractérisé en ce que**

le système d'assistance respiratoire comprend

- un capteur de débit volumique d'inspiration (10) et
- un capteur de débit volumique d'expiration (11),

dans lequel le capteur de débit volumique d'inspiration (10) est configuré pour mesurer une mesure pour le débit volumique [$Vol'(30)$] à travers l'unité de guidage de fluide d'inspiration (30),

dans lequel le capteur de débit volumique d'expiration (11) est configuré pour mesurer une mesure pour le débit volumique [$Vol'(31)$] à travers l'unité de guidage de fluide d'expiration (31),

dans lequel le système d'assistance respiratoire est conçu pour créer au moins temporairement un circuit de respiration entre l'unité de couplage côté patient (9) et l'appareil d'assistance respiratoire (1),

dans lequel le circuit de respiration passe par l'unité de guidage de fluide d'inspiration (30) et par l'unité de guidage de fluide d'expiration (31) et

dans lequel l'appareil de commande (4) est configuré pour déterminer la mesure pour le débit volumique d'inspiration net [$Vol'_{insp}(50)$]

- à l'aide du débit volumique [$Vol'(30)$] mesuré à travers l'unité de guidage de fluide d'inspiration (30) et
- à l'aide du débit volumique [$Vol'(31)$] mesuré à travers l'unité de guidage de fluide d'expiration (31).

8. Système d'assistance respiratoire selon l'une des revendications précédentes, **caractérisé en ce que** l'appareil de commande (4) est configuré pour

- détecter une mesure pour un débit volumique de consigne maximal prédéfini que l'appareil d'assistance respiratoire (1) doit produire en expulsant le mélange gazeux, et
- commander l'élément d'affichage d'inspiration (40) en fonction du quotient entre le débit volumique d'inspiration net [$Vol'_{insp}(50)$] déterminé et le débit volumique de consigne maximal détecté.

9. Système d'assistance respiratoire selon l'une des revendications précédentes, **caractérisé en ce que**

l'indicateur affiché pour le débit volumique d'inspiration net [$Vol'_{insp}(50)$] déterminé comprend une luminosité et/ou une fréquence d'un clignotement ou d'un scintillement de l'élément d'affichage d'inspiration (40),

dans lequel le système d'assistance respiratoire est conçu de sorte que la luminosité et/ou la fréquence dépendent du débit volumique d'inspiration net [$Vol'_{insp}(50)$] déterminé.

10. Système d'assistance respiratoire selon l'une des revendications précédentes, **caractérisé en ce que**

le système d'assistance respiratoire comprend

- une unité de guidage de fluide de dérivation (32),
- un élément d'affichage de dérivation (42) et
- un commutateur (2) pneumatique,

dans lequel l'unité de guidage de fluide de dérivation (32) relie une sortie du commutateur (2) à l'unité de couplage côté patient (9),

dans lequel le commutateur (2) est conçu pour conduire sélectivement un mélange dans l'appareil d'assistance respiratoire (1) ou dans l'unité de guidage de fluide de dérivation (32),
dans lequel l'élément d'affichage de dérivation (42) est fixé

- sur l'unité de guidage de fluide de dérivation (32) ou
- sur une autre unité de guidage de fluide (52) qui est au moins temporairement en liaison fluidique avec l'appareil d'assistance respiratoire (1) et avec l'unité de guidage de fluide de dérivation (32),

dans lequel l'appareil de commande (4) est configuré pour

- déterminer une mesure pour le débit volumique de dérivation qui est le débit volumique à travers l'unité de guidage de fluide de dérivation (32) et
- commander l'élément d'affichage de dérivation (42) en fonction du débit volumique de dérivation déterminé, et

dans lequel l'élément d'affichage de dérivation (42) est configuré pour indiquer, en fonction de la commande, au moins d'une manière perceptible visuellement,

- un indicateur pour la grandeur du du débit volumique de dérivation déterminé et
- éventuellement, un indicateur pour le sens d'écoulement du mélange gazeux à travers l'unité de guidage de fluide de dérivation (32).

11. Système d'assistance respiratoire selon l'une des revendications précédentes,
**caractérisé en ce que**

l'appareil de commande (4) est configuré pour

- détecter une mesure pour un débit volumique d'inspiration net requis à travers l'unité de guidage de fluide d'inspiration (30),

en particulier par une demande à une commande ou à un moyen de régulation de l'appareil d'assistance respiratoire (1), et

- commander l'élément d'affichage d'inspiration (40) en outre en fonction du débit volumique d'inspiration net requis détecté, et

l'élément d'affichage d'inspiration (40) est configuré pour afficher au moins l'un des deux indicateurs en outre en fonction du débit volumique d'inspiration net requis détecté.

**FIG. 1**

FIG. 2

FIG. 3

EP 4 414 013 B1

FIG. 4

FIG. 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 2010242622 A1
- US 2019099578 A1
- DE 102008028662 A1

- US 2022080139 A1
- US 2009143996 A1
- WO 2019154834 A1